# EUROPEAN PATENT SPECIFICATION

(11) **EP 3 927 848 B1**
(45) Date of publication and mention of the grant of the patent: **19.08.2026**
(21) Application number: 20704562.6
(22) Date of filing: 19.02.2020
(51) Int. Cl.: C12Q 1/6886

(54) **PROCESS FOR CLASSIFICATION OF GLIOMA**
VERFAHREN ZUR KLASSIFIZIERUNG VON GLIOMA
PROCÉDÉ DE CLASSIFICATION DE GLIOME

(30) Priority: 22.02.2019 EP 19305215
(43) Date of publication of application: 29.12.2021
(73) Proprietor: Hospices Civils de Lyon, 69002 Lyon (FR); Université Claude Bernard Lyon 1, 69100 Villeurbanne (FR)
(72) Inventor: PONCET, Delphine, 69150 DECINES (FR)
(74) Representative: Regimbeau
(86) International application number: PCT/EP2020/054346
(87) International publication number: WO 2020/169660

(56) References cited:
- JEREMY D HENSON ET AL: "DNA C-circles are specific and quantifiable markers of alternative-lengthening-of-telomeres activity", NATURE BIOTECHNOLOGY, vol. 27, no. 12, 1 December 2009 (2009-12-01), pages 1181 - 1185, XP055048134, ISSN: 1087-0156, DOI: 10.1038/nbt.1587
- BILLARD P. ET AL: "The TeloDIAG: how telomeric parameters can help in glioma rapid diagnosis and liquid biopsy approaches", ANNALS OF ONCOLOGY, vol. 32, no. 12, 1 December 2021 (2021-12-01), pages 1608 - 1617, XP93143402, ISSN: 0923-7534, DOI: 10.1016/j.annonc.2021.09.004
- NOOR H ET AL: "A novel C-circle assay for detecting alternative lengthening of telomeres (ALT) mechanisms", EMBASE, ELSEVIER SCIENCE PUBLISHERS, AMSTERDAM, NL, 1 November 2018 (2018-11-01), XP002793653, DOI: 10.1093/NEUONC/NOY148.164
- FOGLI ANNE ET AL: "Detection of the alternative lengthening of telomeres pathway in malignant gliomas for improved molecular diagnosis", JOURNAL OF NEURO-ONCOLOGY, KLUWER, BOSTON, US, vol. 135, no. 2, 28 July 2017 (2017-07-28), pages 381 - 390, XP036351458, ISSN: 0167-594X, [retrieved on 20170728], DOI: 10.1007/S11060-017-2585-7
- MCDONALD KERRIE L ET AL: "Presence of Alternative Lengthening of Telomeres Mechanism in Patients With Glioblastoma Identifies a Less Aggressive Tumor Type With Longer Survival", JOURNAL OF NEUROPATHOLOGY & EXPERIMENTAL NEUROLOGY, vol. 69, no. 7, July 2010 (2010-07-01), pages 729 - 736, XP002793650, ISSN: 0022-3069
- MANGEREL J ET AL: "Alternative lengthening of telomeres is enriched in, and impacts survival of TP53 mutant pediatric malignant brain tumors", ACTA NEUROPATHOLOGICA 2014 SPRINGER VERLAG DEU, vol. 128, no. 6, 2014, pages 853 - 862, XP002793651, ISSN: 0001-6322
- HAKIN-SMITH V ET AL: "Alternative lengthening of telomeres and survival in patients with glioblastoma multiforme", LANCET, ELSEVIER, AMSTERDAM, NL, vol. 361, no. 9360, 8 March 2003 (2003-03-08), pages 836 - 838, XP004783597, ISSN: 0140-6736, DOI: 10.1016/S0140-6736(03)12681-5
- NGUYEN D N ET AL: "Molecular and morphologic correlates of the alternative lengthening of telomeres phenotype in high-grade astrocytomas", BRAIN PATHOLOGY 2013 BLACKWELL PUBLISHING LTD GBR, vol. 23, no. 3, May 2013 (2013-05-01), pages 237 - 243, XP002793652, ISSN: 1015-6305
- DATABASE EMBASE [online] ELSEVIER SCIENCE PUBLISHERS, AMSTERDAM, NL; 1 November 2018 (2018-11-01), NOOR H ET AL: "A novel C-circle assay for detecting alternative lengthening of telomeres (ALT) mechanisms", XP002793653, Database accession no. EMB-628633441
- NOOR H ET AL: "A novel C-circle assay for detecting alternative lengthening of telomeres (ALT) mechanisms", NEURO-ONCOLOGY 20181101 OXFORD UNIVERSITY PRESS NLD, vol. 20, no. Supplement 6, 1 November 2018 (2018-11-01), pages vi42 CONF 20181115 to 20181118 New Orleans, LA - 23rd Annu, ISSN: 1523-5866
- JEITANY M ET AL: "A preclinical mouse model of glioma with an alternative mechanism of telomere maintenance (ALT)", INTERNATIONAL JOURNAL OF CANCER 20150401 WILEY-LISS INC. USA, vol. 136, no. 7, 1 April 2015 (2015-04-01), pages 1546 - 1558, XP002793654, ISSN: 0020-7136
- JOERG BALSS J ET AL: "Analysis of the IDH1 codon 132 mutation in brain tumors", ACTA NEUROPATHOLOGICA, SPRINGER, BERLIN, DE, vol. 116, no. 6, 5 November 2008 (2008-11-05), pages 597 - 602, XP019657078, ISSN: 1432-0533, DOI: 10.1007/S00401-008-0455-2
- YAN HAI ET AL: "IDH1 and IDH2 mutations in gliomas", NEW ENGLAND JOURNAL OF MEDICINE, THE - NEJM, MASSACHUSETTS MEDICAL SOCIETY, US, vol. 360, no. 8, 19 February 2009 (2009-02-19), pages 765 - 773, XP002538459, ISSN: 1533-4406, DOI: 10.1056/NEJMOA0808710
- LOUIS DAVID N ET AL: "The 2016 World Health Organization Classification of Tumors of the Central Nervous System: a summary", ACTA NEUROPATHOLOGICA, SPRINGER VERLAG, BERLIN, DE, vol. 131, no. 6, 9 May 2016 (2016-05-09), pages 803 - 820, XP035683913, ISSN: 0001-6322, [retrieved on 20160509], DOI: 10.1007/S00401-016-1545-1

## Description

### FIELD OF THE INVENTION

The present invention concerns the classification of tumor brains and the choice of therapeutic options useful for treating patients with tumor brains, based on said classification.

### BACKGROUND OF THE INVENTION

A glioma is a type of tumor deriving from the glial cells of the brain or the spine. Gliomas represent about 30 % of all brain and central nervous system tumors, and 80 % of the malignant brain tumors.

Malignant gliomas are graded from grade II to grade IV, although benign gliomas are designated as gliomas of grade I.

According to the 2016 WHO classification (4^{th} Edition) of tumors of the central nervous system (CNS), summarized in (Louis *et al.,* 2016), malignant tumors of the CNS are classified according to immuno-histological and molecular criteria into different categories:
- diffuse astrocytic and oligodendroglial tumors;
- others astrocytic tumors;
- ependymal tumors;
- choroid plexus tumors;
- neuronal and mixed neuro-glial tumors;
- tumors of the pineal region;
- embryonal tumors (including medulloblastoma, CNS neuroblastoma);
- tumors of the cranial and paraspinal nerves; and
- "other" gliomas.

Gliomas are defined as tumors of the category "diffuse astrocytic and oligodendroglial tumors". In this 4^{th} edition of the WHO classification, gliomas are defined with molecular biomarkers, and then subclassed into the following subclasses: the oligodendroglioma (OD), the astrocytoma (A), the glioblastoma (GBM), the diffuse midline glioma and the oligoastrocytoma.

Molecular features define theses subclasses:
- Complete deletion of both the short arm of chromosome 1 (1p) and the long arm of chromosome 19 (19q), designated as 1p/19q co-deletion, is the molecular genetic signature of oligodendrogliomas;
- Histone mutations *(H3.3 or H3.1* K27M mutation and G34R/V H3.3 mutation) have been identified in diffuse midline glioma; presence of a histone mutation was recognized to portend an adverse prognosis, regardless of the histological grade of the lesion;
- Isocitrate dehydrogenase 1 or 2 mutations are observed in low-grade astrocytoma (anaplasic astrocytoma (AA) or diffuse astrocytoma (AD)), oligodendroglioma and secondary GBM, also named IDH mutated GBM (IDHmt). Primary GBM do not show mutation of IDH, and are classified as IDHwt GBM.

When no molecular marker is available, glioma are classified as being "NOS" (for Not Otherwise Specified). In this circumstance, glioma is designated with the term "oligoastrocytoma".

Histological criteria such as vascularization, necrosis or proliferation further refine each subclass into different grades: diffuse (grade II) or anaplasic (grade III) glioma. Grade IV, the most advanced stage, defines glioblastoma.

Because of their diffusely infiltrating nature, grade II to IV gliomas cannot be completely resected and are not curable by surgical excision.

Each subclass of glioma is defined by a median time of overall survival (OS) of the patients affected by said glioma. A brief summary of features of each subclass is presented below.
1. Oligodendrogliomas are believed to originate from the oligodendrocytes of the brain or from a glial precursor cell. They occur primarily in adults (median age at onset is 45 years) but are also found in children. Oligodendrogliomas are slowly growing, and therefore patients have a prolonged survival compared to other glioma. They benefit from less aggressive therapeutic approach. Median survival times for patients affected with oligodendroglioma are of about 15 years for grade II, and 3.5 years for grade III.
2. Astrocytomas (grade II and III) can occur in most parts of the brain. They originate in a particular kind of glial cells, star-shaped brain cells called astrocytes. People can develop astrocytomas at different ages. For grade II to IV astrocytomas, despite decades of therapeutic research, curative treatment is still non-existent. The median overall survival is of about 5 to 8 years for grade II astrocytoma, and about 3 years for grade III astrocytoma.
3. Grade IV astrocytoma is designed as "multiform glioblastoma" (GBM). It is the most frequent adult brain tumor, and one of the most aggressive tumors among all human cancers. The extremely infiltrative nature of this tumor makes complete surgical removal impossible.

Among glioblastoma, clinicians distinguish primary glioblastoma that have an IDHwt status, and secondary glioblastoma that present a mutation in IDH 1 and/or IDH 2 (IDHmt) and that are supposed to derive from low-grade, IDHmt astrocytoma.

Patients affected with a secondary glioblastoma present a better median overall survival than those affected with primary glioblastoma: the overall survival of patients with primary glioblastoma ranges from 7 to 15 months, whereas the overall survival of patients with secondary glioblastoma is about 2 years and a half.

Diffuse astrocytoma IDHmt (grade II), anaplasic astrocytoma IDHmt (grade III), glioblastoma IDHmt (grade IV), glioblastoma IDHwt (grade IV) are mainly reported in adults.
4. Diffuse midline glioma are grade IV tumors that tend to occur in children and young adult; they are characterized with the presence of a histone mutation. Diffuse midline glioma tends to spread out and invade neighboring tissue. These tumors are associated with an overall dismal prognosis, with a median overall survival of less than 12 months.
5. Oligoastrocytoma is a subclass of gliomas that present with an appearance of mixed glial cell origin, astrocytoma and oligodendroglioma. This class is considered to be obsolete by several scientifics, since it designates morphologically ambiguous tumors that should actually be classified into astrocytomas or oligodendrogliomas. With the identification of the molecular biomarker "co-deletion of 1p/19q", these gliomas can now be classified into one the following category:
   a. Oligodendroglioma if the co-deletion is present;
   b. Astrocytoma if no co-deletion is identified.

Histopathological classification is the basis of the World Health Organization (WHO) classification; however, it suffers from a high variability of interpretation from one practician to another. This is also due to the fact that tumors from the same category are highly heterogeneous. Consequently, therapeutic strategies may be wrongly chosen, if the glioma is incorrectly classified.

Recently, IMPACT-NOW, the Consortium to Inform Molecular and Practical Approaches to CNS Tumor Taxonomy, has produced additional guidelines for glioma sub-classification. These guidelines designate as « NEC » (Not Elsewhere Classified) tumors with histo-molecular characteristics that cannot be classified according to the WHO 2016 diagnosis (for example, because of a mismatch between clinical, (immuno)histological and/or genetic features) ¹⁻⁴.

Among NEC, the "astrocytoma IDHwt" subgroup consists in grade II and III glioma with astrocytoma features (based on morphological and immuno-histological parameters) but without any IDH mutation.

This "astrocytoma IDHwt" subclass is particularly heterogeneous in terms of response to treatment and overall survival, with a much more severe prognosis than the IDH mutated astrocytoma. The 3rd update of the IMPACT-NOW consortium advises to class these tumors as "Diffuse astrocytic glioma, IDH-wildtype, with molecular features of glioblastoma, WHO grade IV" if any of the following markers are observed: EGFR amplification, whole chromosome 7 gain and whole chromosome 10 loss (+7/-10) or TERT promoter mutation. If none of these markers is retrieved, no guideline is available for the treatment of said "NEC" tumors.

Beside histological features and specific molecular biormarkers, an additional defining feature for gliomas is the characterization of the telomere maintenance mechanisms (TMMs).

Telomeres are DNA-protein complexes present at the end of chromosomes in eukaryotic cells, which play a crucial role in cellular survival. Indeed, in healthy cells, a gradual shortening of telomere happens at each replicative cycle. When telomeres reach a critical size, cells stop to proliferate and enter in senescence. Most cancer cells maintain the length of their telomeres by the reactivation of telomerase, or through the telomerase-independent alternative lengthening of telomeres (ALT) mechanism.

A retrospective study of 573 glioblastoma patients showed that ALT+ glioma patients had longer survival, which suggests that most ALT+ tumors may be less aggressive gliomas (McDonald *et al.,* 2010). Another prognosis studies were realized on high-grade astrocytomas (Nguyen *et al.,* 2012).

(Mangerel *et al.,* 2014) and (Fogli *et al.,* 2017) teach the use of a C-circle assay for determining the ALT status of human glioma samples. Both articles demonstrate that this specific C-circle assay is useful for the detection of ALT activation.

(Hakin-Smikth *et al.,* 2003) reports the analysis of both telomerase activity (ALT status) and telomere lengths in tumors from 77 patients having gliobastoma multiforms, of primary or secondary types.

In view of the discordant findings from these studies, the prognosis value of the ALT status is still uncertain. In particular, a glioma classification method taking into account the ALT status of the tumor, among other parameters, is still to be defined.

The international application WO 2017/127803 proposes a glioma classification method based on multiple molecular features, comprising:
i) IDH mutation status,
ii) DNA methylation cluster,
iii) RNA cluster,
iv) Telomere length,
v) Telomere maintenance, and
vi) At least one biomarker, in particular selected among the following biomarkers: an amplification of Epidermal Growth Factor Receptor, a mutation in the protein p53, an IDH mutation, the co-deletion 1p19q, a chromosome 7 amplification coupled with a chromosome 10 deletion, a Cyclin-dependent kinase 4 (CDK4) amplification coupled with a Cyclin Dependent Kinase Inhibitor 2A (CDKN2A) deletion, a chromosome 19 (chrl9) amplification coupled with a chromosome 20 (chr20) amplification, a B-raf gene mutation coupled with a Neurofibromin 1 (NF1) mutation.

This method is based on the measure of at least six parameters, including molecular parameters. In view of (i) the quantity of biological material, (ii) the high cost and (iii) the time-consuming techniques that are necessary for measuring these parameters, this classification process would not be feasible in an usual clinical practice. Moreover, the timing for choosing a therapeutic strategy would be too long in regard to the duration of this diagnosis process.

A relevant classification of gliomas is the key for improving the therapeutic strategies and hopefully the clinical outcomes.

Currently, hospital services classify gliomas according to immuno-histological features and on the basis of available molecular biomarkers (such as mutation of TERT, mutation of histones, mutation of IDH, amplification of EGFR, deletion of CDKN2A). This classification process is fastidious, expensive, and variable from one hospital to another, since highly dependent on the pictures interpretation of the pathologist and on the molecular biology facilities available in the hospital.

Since the current classification process is not satisfying, active research is currently conducted in order to identify and classify as precisely as possible gliomas, in a clinical environment, for helping health practitioners to choose the best therapeutic approach for patients.

Ideally, an efficient classification process would answer to the following criteria:
- It would be based on reproducible and reliable techniques;
- It would give homogeneous classification when performed by different services or hospitals;
- It could be realized with a few amount of DNA and, if necessary, with a poor quality sample of DNA;
- It would allow the classification of outsiders tumors, with no specific features, currently classified as "oligoastrocytoma NOS" (not otherwise specified), NEC for "Not elsewhere classified" or "IDHwt astrocytoma"; and
- It would be easy, cheap and rapid, to give a quick answer to the clinician.

The present invention discloses a process of classification of gliomas that present all the advantages listed above.

### SUMMARY OF THE INVENTION

The present invention relates to an *in vitro* process for classifying a glioma, comprising the following steps:
a. Measuring at least, from a glioma patient biological sample, the Alternative Lengthening of Telomeres (ALT) status of said glioma;
b. Optionally, determining the isocitrate dehydrogenase genes mutation status (IDH status) of said glioma;
c. Based on the data obtained in steps (a) and optionally (b) and, if available, on the histological grade of said glioma, classifying said glioma in one of the five following classes: oligodendroglioma-like, glioblastoma IDHwt-like, glioblastoma IDHmt-like, low-grade astrocytoma-like and other gliomas.

Among said five classes, gliomas of low grades may be further refined as being of grade II or III, as a function of the histological grading, if available/known.

Furthermore, gliomas of the class "low-grade astrocytoma-like" may be subclassified as belonging to one of the two subclasses designated as t-low grade Astrocytoma (tLGA) and t-Astrocytoma grade IV (tA-IV).

In another specific implementation of this process, the ALT status in (a) is measured by performing a C-circle assay coupled to a telomere-specific PCR (TeloPCR), thereby obtaining a C-circle value and selecting the ALT status of the glioma after comparison of said C-circle value to threshold values, determining several classes, for example four classes : "ALT-", "ALT intermediate", "ALT+", and "ALT++".

In another specific implementation of this process, the ALT status in (a) is measured by performing a C-circle assay coupled to a telomere-specific PCR, thereby obtaining a C-circle value and selecting the ALT status of the glioma after comparison of said C-circle value to threshold values, determining three classes:
- ALT++, when the C-circle value is greater than the threshold value "high";
- ALT+, when the C-circle value is greater than the threshold "positive";
- ALT-, when the C-circle value is less than the threshold value "positive".

In another specific implementation of this process, step (a) further comprises the measure of the telomere length status of said glioma.

In this specific implementation of this process, the telomere length status is measured by quantifying the telomeric DNA with a telomere-specific PCR, thereby obtaining a T-length value and selecting the telomere length status of the glioma after comparison of said T-length value with threshold values determining the 3 following classes: "short", "intermediate" and "long".

The present invention also concerns the use of the *in vitro* process as described herein for reclassifying a glioma of the group "astrocytoma IDHwt" or of the group "NEC", according to the WHO's classification, in one of the following classes: oligodendroglioma-like, glioblastoma IDHwt-like, glioblastoma IDHmt-like, t-low grade Astrocytoma (tLGA) and t-Astrocytoma grade IV (tA-IV), and other gliomas

Among said classes, gliomas of low grades may be further refined as being of grade II or III as a function of the histological grading, when available.

The present invention also concerns a process for choosing a therapeutic strategy for treating a glioma, comprising the steps of:
a) Implementing the *in vitro* process for classifying said glioma as described above; and
b) Based on said classification, choosing the more adapted therapeutic strategy for the patient affected by said glioma.

The present invention also concerns a process for adapting a therapeutic strategy for treating a glioma, comprising the steps of:
a) Implementing the *in vitro* process for classifying said glioma as described above; and
b) Based on said classification, adapting the therapeutic strategy for the patient affected by said glioma.

The present invention also concerns a computer program product comprising code instructions for implementing a process as defined above, for classifying a glioma.

The present invention also concerns a kit for the implementation of the processes as described above, comprising:
- Reagents suitable for performing a C-circle assay;
- Reagents suitable for performing a Telomere-specific PCR; and
- Adequate internal controls comprising genomic DNA from ALT+ cells and from ALT- cells,
wherein both substeps of measure of C-circle and telomere length are concomitantly performed by one duplex PCR (dTeloPCR).

The present invention also concerns an inhibitor of the telomere maintenance mechanism for its use in the treatment of a glioma, wherein said glioma has been previously classified according to the process as described above.

### BRIEF DESCRIPTION OF THE FIGURES

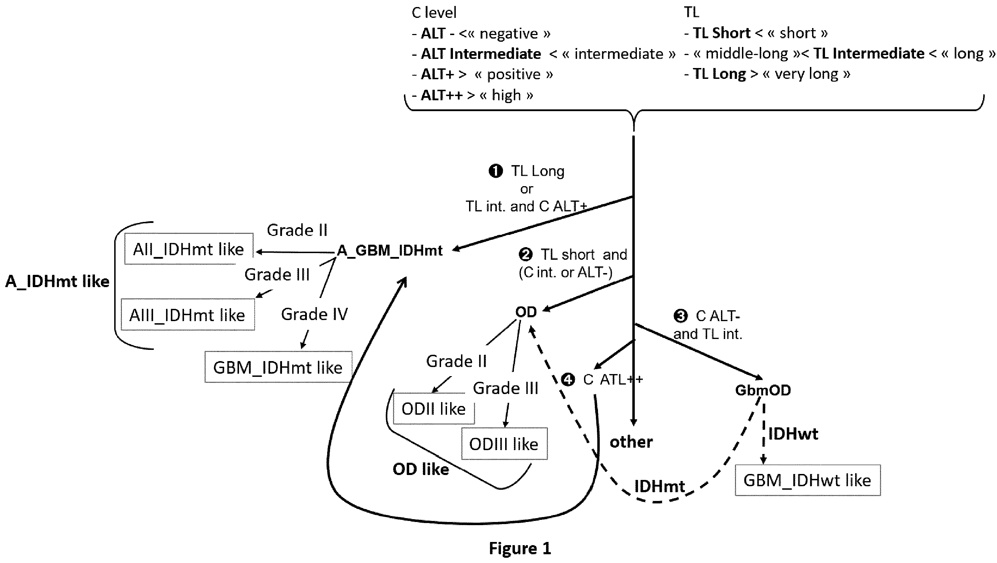

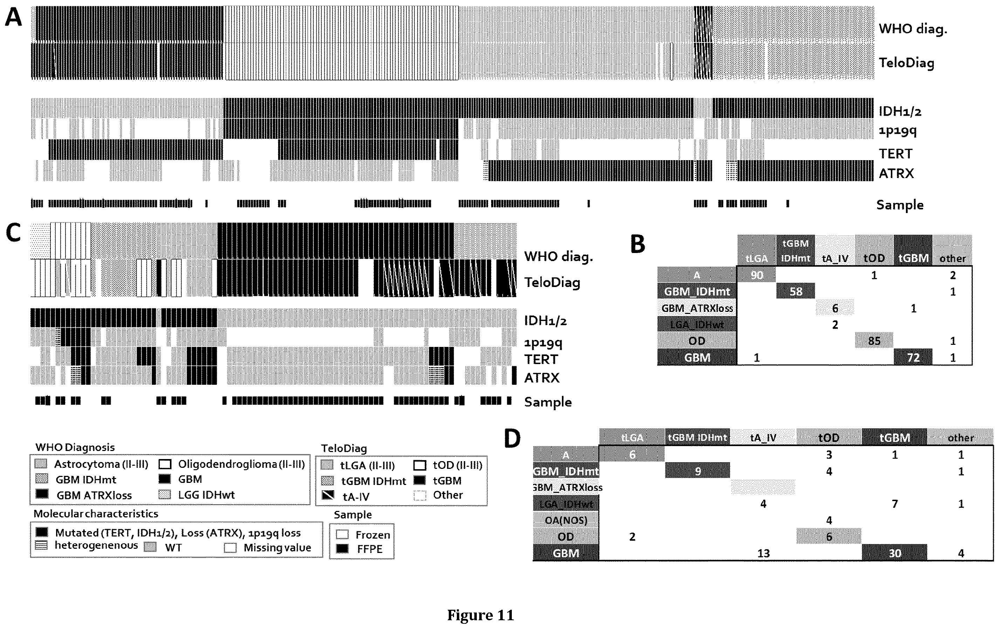

**Figure 1****. Schematic representation of the classification steps**
   The following abbreviations are used:
   TL: T-length value; long, int. and short designate the three possible TL status;
   C: C-circle value; int.: intermediate; ALT++, ALT+, ALT int. and ALT- designate the four possible ALT status;
   IDH: IDH status is chosen among IDHwt (no mutation) or IDHmt (at least one mutation in IDH1 and/or IDH2)
   Classification of gliomas "like" according to the algorithm of the invention: OD : oligodendroglioma; OD II: oligodendroglioma grade II; OD III: oligodendroglioma grade III; GbmOD: GBM or OD; GBM_IDHwt : primary glioblastoma; GBM_IDHmt: secondary glioblastoma; A_GBM_IDHmt: astrocytoma or secondary glioblastoma (mutated for IDH); A_IDHmt: astrocytomas; All_IDHmt : astrocytoma grade II; Alll_IDHmt : astrocytoma grade III.
**Figure 2****. Schematic representation of the classification steps for an astrocytoma IDHwt**
   The same abbreviations than in figure 1 are used.
**Figure 3****. Standard curve for calculation of qPCR efficiency**
   qPCR were led using DNA from an ALT positive tumor (left : TeloPCR, right : qPCR targeting the reference housekeeping gene 36B4).
   (A) representative amplification curves are shown with (+) or without (-) pre-incubation with the enzyme ϕ29, as shown by the corresponding arrows. Telomere length is determined in the ϕ29-, and the c-circle value is calculated by using +ϕ29 and - ϕ29 values. Note that the same amount of genomic DNA is used in the two conditions (same curves for 36B4).
   (B) DNA from an ALT positive tumor was diluted and the CT value (y) is plotted against the log-transformed dilution factor (x) (TeloPCR on the left, and qPCR against 36B4 on the right). Note that the correlation coefficient (R²) is nearly 1 for both qPCR.

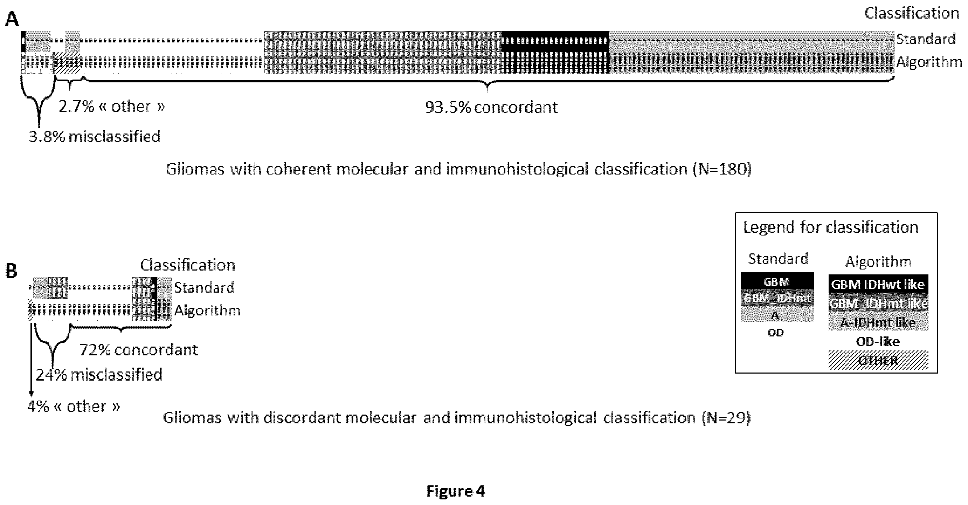

**Figure 4****. Comparison of the diagnosis value of the two classifications**
   Gliomas (with the exception of anaplastic astrocytoma IDHwt) have been classified according to the standard algorithm according to the WHO 2016 classification (upper line), or with the algorithm of the invention (bottom line).
   The algorithm of the invention allows a separation into five subclasses: "oligodendroglioma-like", "glioblastoma IDHwt-like", "glioblastoma IDHmt-like", "low-grade astrocytoma -like" and "other" (the code is depicted in the legend).
      (A) Only patients with molecular biomarkers in agreement with the immuno-histochemical classification are analyzed (N=180), the two classifications are concordant for 93.5% of gliomas, are different (noted misclassified) for 3.8%, and 2.7% of gliomas are classified as "others".
      (B) Considering only the gliomas with discordant molecular biomarkers and immuno-histochemical classification (N=29), the algorithm is in agreement with histological classification for 72% of gliomas.

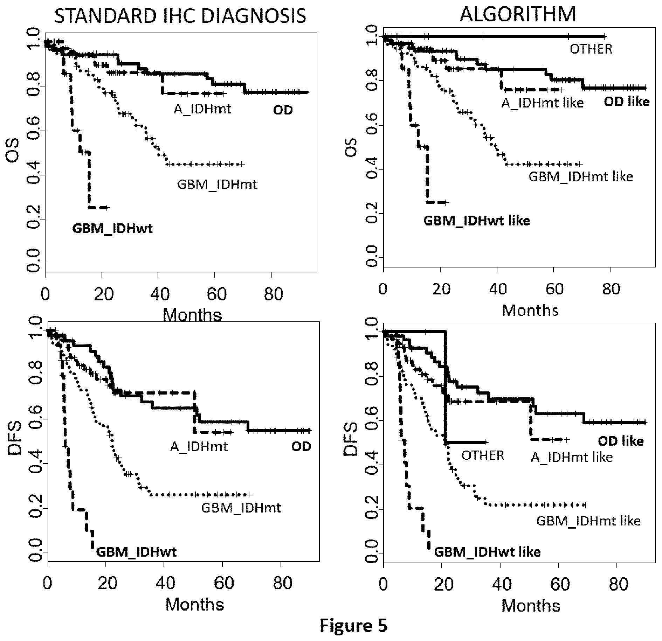

**Figure 5****. Comparison of the results curves of median overall survival and median disease-free survival obtained after standard classification and classification according to the process of the invention**
   Gliomas (with the exception of anaplastic astrocytoma IDHwt) have been classified according to the standard algorithm following the WHO 2016 classification (left curves), or with the algorithm of the invention (rigth curves).
   The overall survival (OS, upper curves) and the disease free survival (DFS, bottom curves) have been followed for each patient. 210 patients were analyzed (Standard classification: 74 low-grade astrocytoma, 24 glioblastoma IDHwt, 57 glioblastoma IDHmt, 55 oligodendroglioma). Among these patients, 29 have discordant molecular parameters as regard with the immuno-histological classification.
   The algorithm of the invention allows a separation into five subclasses: oligodendroglioma-like, glioblastoma IDHwt-like, glioblastoma IDHmt-like, low-grade astrocytoma -like (A_IDHmt like), and other gliomas.
   The following abbreviations are used:
      OD: oligodendroglioma; A_IDHmt: Astrocytoma_IDHmt; GBM_IDHmt: secondary glioblastoma (also called glioblastoma IDHmt); GBM_IDHwt: primary glioblastoma (also called glioblastoma IDHwt).
      Note that the curves are highly similar regarding the overall survival, and that the algorithm is even more discriminant that the standard classification considering the low grade astrocytoma and the oligodendroglioma.

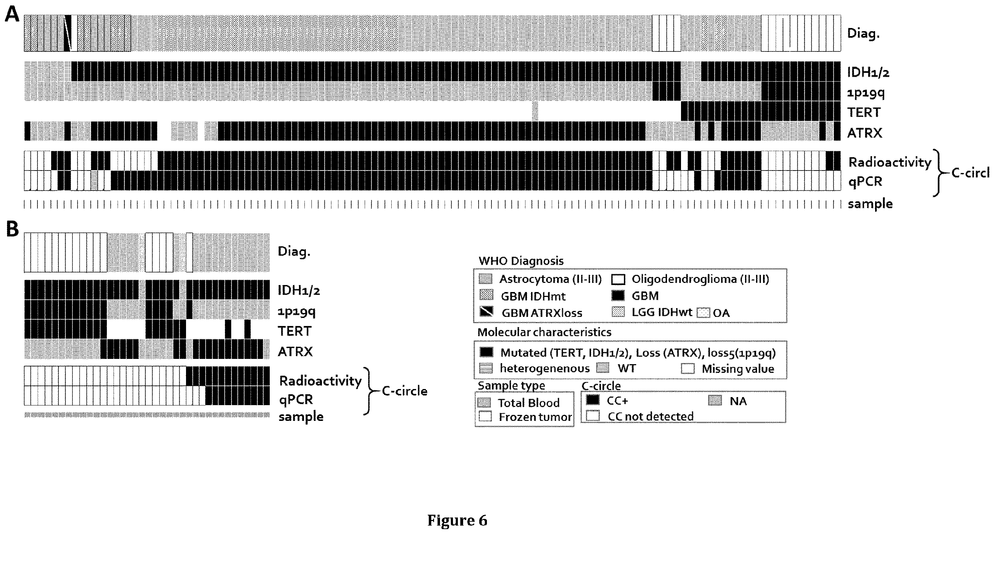

**Figure 6****. The duplex TeloPCR gives concordant result as compared to the reference technic.**
   Results obtained by radio-activity determination or by multiplexe teloPCR have been compared in 120 frozen tumors (A) and in 37 total blood sample (B). Characteristics of the tumors are depicted regarding the diagnosis and the molecular features.

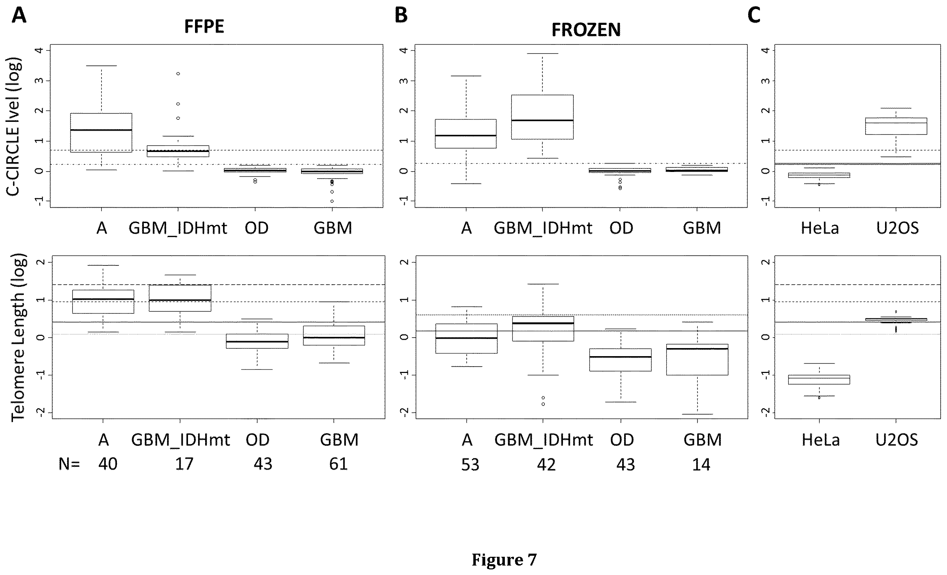

**Figure 7****. Gliomas show different telomeric features as a function of subtypes.**
   C-circle level and telomere length have been determined by the dTeloPCR method, on 161 FFPE samples (A) and 152 frozen samples (B), as a function of glioma subtype (A :astrocytoma, GBM_IDHmt, GBM: glioblastoma, OD: oligodendroglioma). Control DNA from a TERT positive cell line HeLa or ALT positive cell line U2OS were used in each experiment (N=40 independent determination), results are shown (C).

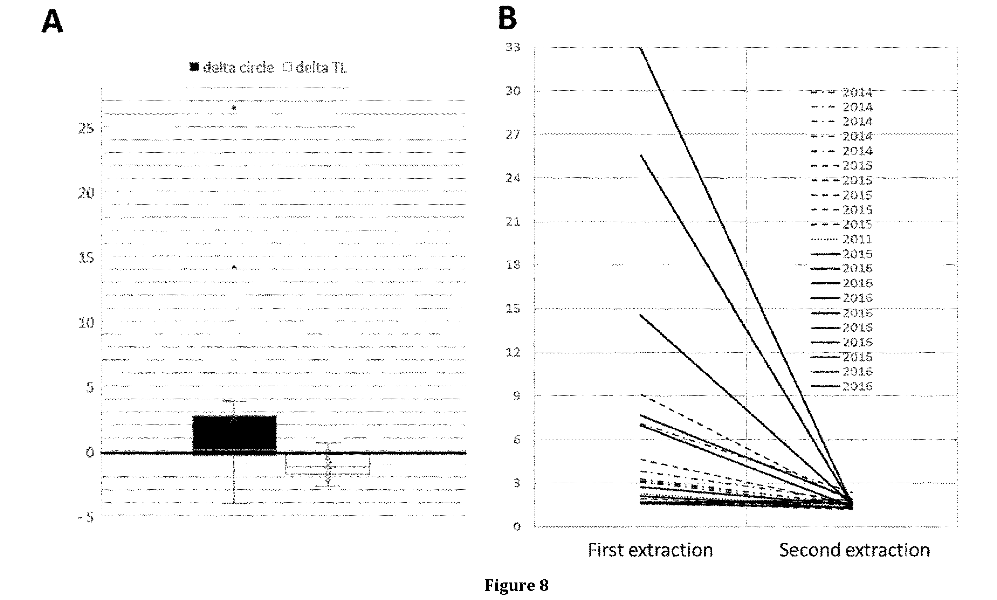

**Figure 8****. Sample preparation and conservation modalities impact the measurement of the telomeric parameters (dTEloPCR).**
   (A) CC (C-circles) and TL (telomere length) have been determined in paired FFPE/frozen samples from 16 patients. The differences between CC reported in frozen minus FFPE is depicted (delta circle). The same has been calculated for TL (delta TL). 0 stand for no difference.
   (B) DNA was re-extracted from 21 FFPE samples of ALT+ gliomas 3 (2016) to 8 years (2011) after their inclusion in FFPE. The c-circle content is compared with those obtained by analyzing DNA extracted at the time of initial diagnosis.

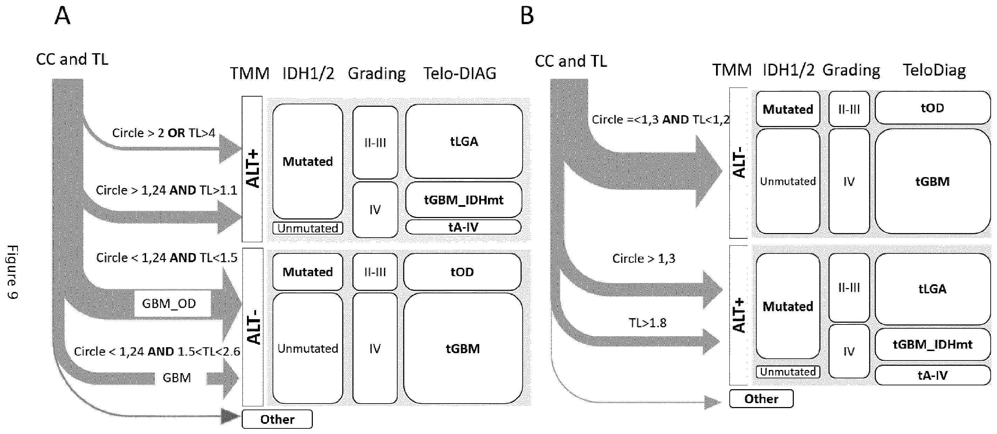

**Figure 9****. Detailed steps and thresholds of the algorithm**
   A) Classification of FFPE tumors. B) Classification of frozen tumors. As a function of CC level and TL values, tumors are separated into two TMM subgroups : ALT + or ALT-. The determination of IDH1/2 mutational status and grading allows to further classify the tumors in five subgroups (tOD, tGBM, tLGA, tGBM_IDHmt and tA-IV). Unclassified tumors are designated as "Other".

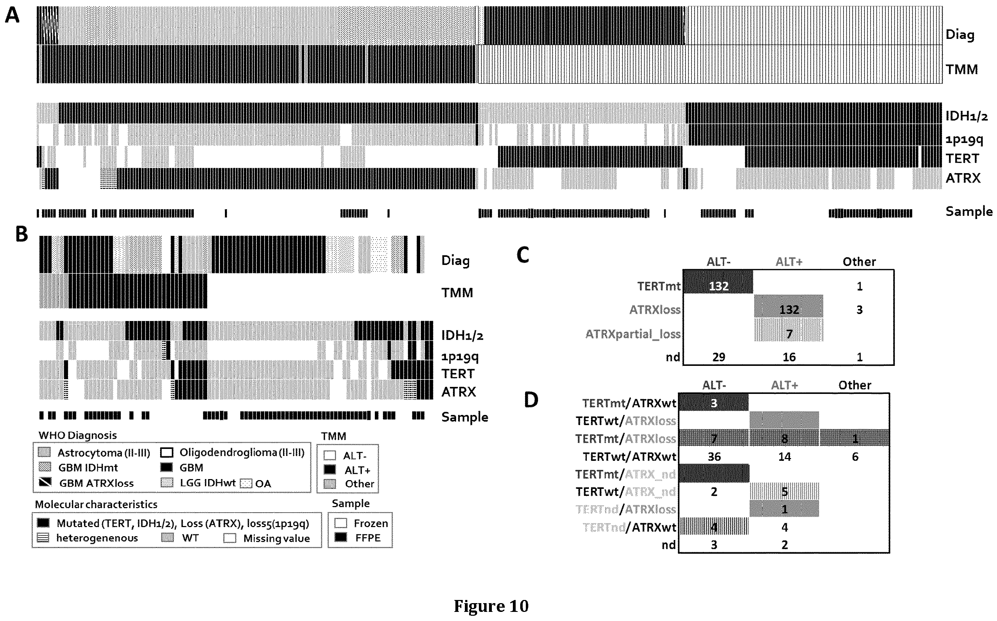

**Figure 10****. Concordance of the TMM determined by the first step of the algorithm with TERT and ATRX status.**
   Gliomas were assessed for C-circle by the dTeloPCR method, and classified in ALT+ an ALT- subgroups according to the first step (a) of the process/algorithm (TMM).
   (A, C) 321 gliomas with molecular markers (IDH1/2, 1p19q deletion, TERTmt and ATRXloss) in agreement with the integrated WHO 2016 diagnosis (WHO diagnosis) are depicted. We observe a perfect concordance between the ALT+ subgroup and the ATRXloss, so as for the ALT- subgroup and TERT mutation.
   (B, D) 96 tumors with discordant molecular features were next analyzed. Only 7 were not classified, all the other tumors were assigned to ALT+ or ALT- subgroups. nd:ND
**Figure 11****. Comparison of the WHO 2016 classification and the TeloDiag classification.**
   (A,B) C-circle (dTeloPCR method) assays were performed on 321 tumors (frozen or FFPE) with coherent molecular and immune-histochemical markers. Classification of these tumors by the TeloDiag is compared to the WHO 2016 based classification. A diagram recapitulating the correspondence is shown. (C,D) The same analysis was done on 96 tumors with discordant molecular biomarkers.

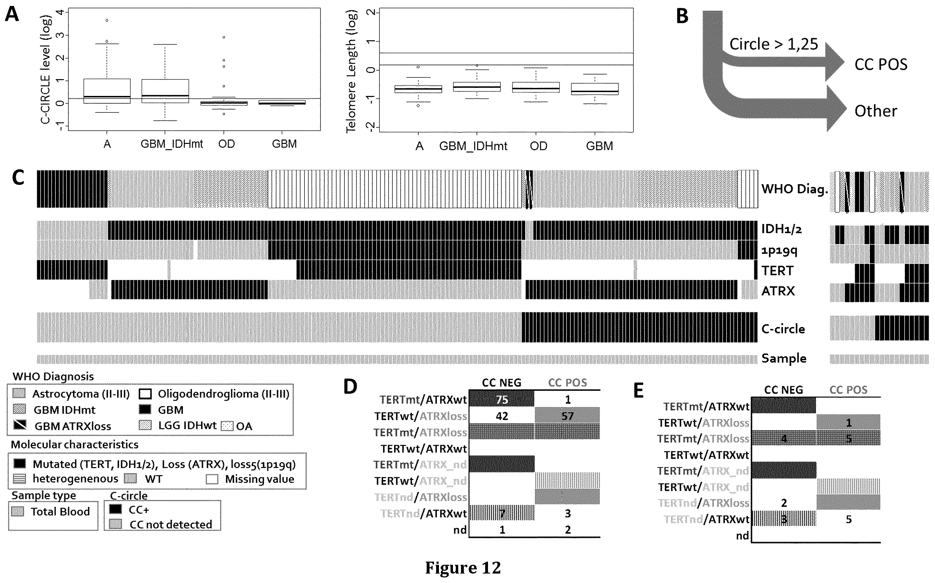

**Figure 12****. Detection of C-circle in total blood sample by the dTeloPCR method.**
   C-circle assays were performed on 208 blood samples.
   (A) results of C-circle detection and TL determination are shown as a function of glioma subtype, no difference regarding TL in blood DNA was noted (as expected) and a higher level of C-circle in blood from patients with ALT tumor (Astrocytoma, GBM-IDHmt) was observed.
   (B) A cut-off value of 1.25 was used to classify the sample as C-circle positive (CC+) and C-Circle negative (CC not detected), whatever the TL result were.
   (C-E) Detection of CC in total blood samples from patients with tumor showing concordant molecular and immune-histological markers (N=188) (C on the left, E), or bearing tumors with discordant molecular markers (NEC, N=20) (C on the right, D). (D-E) Summary of CC results as a function of gliomas subtype are shown.

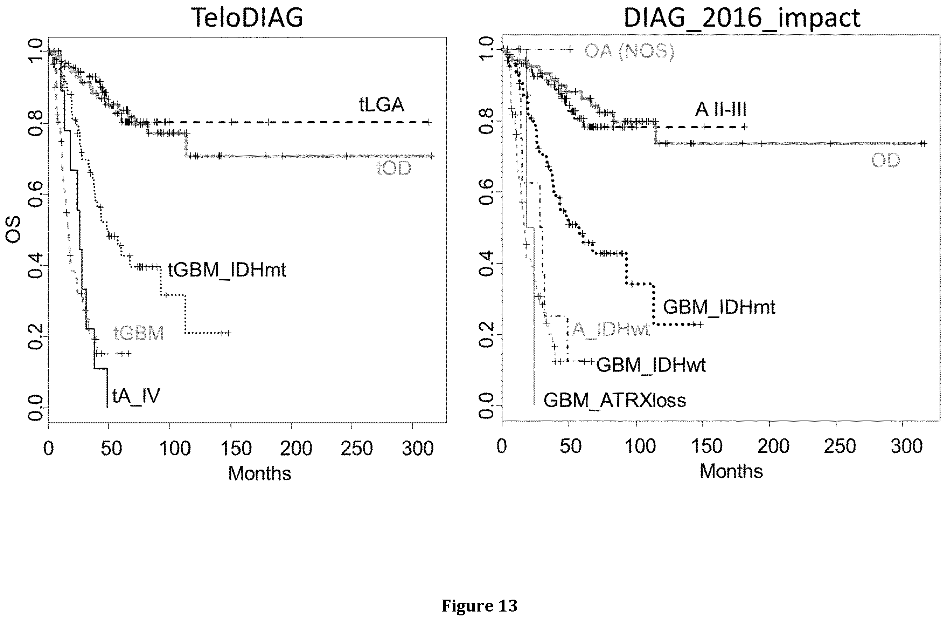

**Figure 13****. Prognosis significance of the TeloDiag classification.**
   Survival curves of N=307 gliomas (63 GBM IDHwt, 4 GBM ATRXloss, 65 GBM_IDHmt, 100 Astrocytoma IDHmt (A II-III), 2 oligoastrocytoma (OA), 64 oligodendroglioma (OD)), are shown following the WHO 2016 classification (with c-Impact-now updates). The same patients classified following the TeloDIAG were analyzed in the same way.
   The TeloDIAG has the advantage to reduce the number of subgroups and to keep the same prognosis significance. Note that the crossing of the tLGA and tOD curves (not expected), is due to fewer disease events in these two subgroups (13 over 91 for the tLGA and 15 over 75 for the tOD).

### DETAILED DESCRIPTION OF EMBODIMENTS OF THE INVENTION

"Glioma" is identified on the basis of histopathological analysis. Classification as a "glioma" is given by a health practician on the basis of:
- morphological criteria, such as cell shape and density, nuclear atypias, vascularisation, necrosis and mitosis number (determined on hematoxilin-eosin-labelled sections);
- immuno-histochemical labelling of different epitopes, such as GFAP, OLIG2, IDH1-R132H, ATRX, TP53, Ki67, and
- if available, molecular biomarker such as mutations of IDH1 and 2, and/or 1p/19q deletion (according to the WHO classification).

The present invention relates to an *in vitro* process for classifying a glioma, comprising the following steps:
a. Measuring at least, from a glioma patient biological sample, the Alternative Lengthening of Telomeres (ALT) status ;
b. Optionally, determining the isocitrate dehydrogenase genes mutation status (IDH status) of said glioma;
c. Based on the data obtained in steps (a) and optionally (b) and, if available, on the histological grade of said glioma, classifying said glioma in one of the five following classes: oligodendroglioma-like, glioblastoma IDHwt-like, glioblastoma IDHmt-like, low-grade astrocytoma-like and other gliomas.

This classification process allows the distinction of subclasses of gliomas, different from the 2016 WHO standard classification of tumors of the central nervous system.

The subclasses proposed by the present invention are the following:
- oligodendroglioma-like,
- glioblastoma IDHwt-like (also designated as primary glioblastoma),
- glioblastoma IDHmt-like (also designated as secondary glioblastoma),
- low-grade astrocytoma like, and
- other gliomas.

The phrase "low-grade astrocytoma" designates astrocytoma of grade II (diffuse astrocytoma) and of grade III (anaplastic astrocytoma). Nevertheless, it was found that this class "low-grade astrocytoma -like" actually includes two subclasses designated as t-low grade Astrocytoma (tLGA) and t-Astrocytoma grade IV (tA-IV).

Therefore, in a specific implementation of the invention, the *in vitro* process for classifying a glioma comprises the following steps:
a. Measuring at least, from a glioma patient biological sample, the Alternative Lengthening of Telomeres (ALT) status of said glioma;
b. Optionally, determining the isocitrate dehydrogenase genes mutation status (IDH status) of said glioma;
c. Based on the data obtained in steps (a) and optionally (b) and, if available, on the histological grade of said glioma, classifying said glioma in one of the six following classes: oligodendroglioma-like, glioblastoma IDHwt-like, glioblastoma IDHmt-like, t-low grade Astrocytoma (tLGA) and t-Astrocytoma grade IV (tA-IV), and other gliomas.

The term "like" is used to mean that the patient in the group is expected to have similar response to treatment and overall survival as the group's name, but not necessarily have the corresponding immuno-histological or molecular parameters.

Each group of patients having glioma is defined by a median time of overall survival of said patients. For example, a patient whose glioma is classified in the class "glioblastoma IDHwt-like" can expect an overall survival time comprised between 7 and 15 months.

Advantageously, this classification process allows the re-classification of some gliomas that had been previously wrongly characterized, and consequently an incorrect expectation of median time of disease free survival and/or of overall survival had been announced to the patient. Furthermore, a non-adapted therapeutic strategy might be used for incorrectly classified patients.

In this case, the algorithm of the invention would allow to inform the patient that his/her median overall survival time would be 7 to 15 months (GBM IDHwt-like), instead of 15 years for an oligodendroglioma of grade II.

Also, the therapeutic strategies will not be the same, depending on the classification conclusions.

As presented in figure 4, the process of the invention allows a classification with a prognostic value at least equivalent to the standard (WHO's) classification.

Advantageously, this classification process is easy to implement in a clinical environment, since it necessitates only DNA extracted from said glioma sample, for the measure of parameter in step (a) and optionally (b).

Optionally, the grading of the tumor is measured on the basis of immuno-histochemical analyses of the glioma sample. Preferentially, the grading of the tumor is previously known from the anatopathologic analysis.

It is understood that the process according to the invention comprises at least two steps (a) and (c) but might also comprises other supplementary steps, well known by the man skilled in the art, such as the optional step (b), and also the detection of an EGFR amplification, the detection of a CDKN2A copy number loss, and/or the detection of chromosomal abnormalities like the gain in chromosome 7p correlated with loss of chromosome 10q (Inda et al., 2003).

In a specific embodiment of the invention, the process of the invention comprises both steps (a) and (c).

In a specific embodiment of the invention, the process of the invention comprises the three steps (a), (b) and (c).

In a specific embodiment of the invention, the process of the invention consists in both steps (a) and (c).

In a specific embodiment of the invention, the process of the invention consists in three steps (a), (b) and (c).

### Origin of the DNA for the measure of parameters in steps (a) and (b)

Surgery to remove as much of the tumor as possible is usually the first step in treating gliomas. After this procedure, a sample of the tumor is further analyzed.

If no surgery is performed, a stereotaxic biopsy may be realized to obtain a tumor sample. From this tumor sample, DNA is extracted by any technique well known by the man skilled in the art.

In an embodiment of the invention, the parameters ALT status and IDH status are measured on the basis of DNA extracted from a sample of said glioma, that has been recovered by any technique known by the man skilled in the art.

In most cases, glioma samples are not analyzed directly but are conserved before extraction of DNA. Typically such collected tissue samples are frozen or are processed as paraffin blocks. Advantageously, these tumor samples are annotated with clinical information on the patient.

In an embodiment of the invention, the parameters ALT status and IDH status of tumor samples are measured on the basis of DNA extracted from a glioma sample that has been conserved in paraffin or frozen.

Advantageously, the techniques for measuring parameters ALT status and IDH status of tumor samples can be realised on the basis of DNA extracted from a glioma sample that has been conserved in paraffin or frozen, even if said DNA is of poor quality and/or in a small amount.

Frozen tissues show a higher level of C-circles but a lower level of telomeric sequence amplification, thereby producing a bias toward a lower TL evaluation (Fig.8A).

The FFPE samples (conserved in paraffin) are thus preferred, but should be extracted during the first year following the biopsy/surgery, to avoid the degradation of C-circle due to prolonged FFPE conservation and the risk of false negative (Fig.8B)

According to a preferred embodiment of the invention, the step (a) of the process further comprises the measure of the telomere length status of said glioma. Technique for this measure is described in a later chapter of the specification.

### Implementation of the process on DNA extracted from blood of cerebrospinal fluid of a patient with glioma

In an aspect of the invention, the parameters ALT status and optionally IDH status of tumor samples are measured on the basis of DNA of glioma cells, extracted from a blood sample or a cerebrospinal fluid sample from a patient having said glioma.

Glioma cells have a tendency to necrose and cell debris and/or free DNA circulate in blood and cerebrospinal fluid of patients affected by glioma. Moreover, cells designated as Circulating tumor cells (CTCs) issued from a primary tumor are able to circulate around the body in the blood circulation. From circulating DNA (circDNA) and/or CTCs, tumor-specific DNA can be purified and analyzed in order to assess the parameters ALT status and IDH status.

In this aspect of the invention, the term "glioma sample" designates the DNA from the glioma cells, that is obtained from the blood or from the cerebrospinal fluid of the patient.

In the sense of the invention, "blood sample" designates a sample comprising all types of blood cells (white cells, red cells and platelets). Peripheral white cells consist of lymphocytes (T cells, B cells, NK cells) monocytes and polynuclear cells. After total centrifugation of blood, all blood cells are collected to obtain the "blood sample" of the invention. This blood sample is expected to comprise circulating tumoral cells (CTC).

This implementation of the process on a blood sample is advantageous since, in the preliminary steps not included in the process, the invasive step of biopsy for obtaining a tumor sample is avoided.

A disadvantage of the use of a blood sample is that mutated DNA from CTC is drowned in a huge amount of "normal" DNA from other blood cells (for IDH1/2), whichwould prevent from establishing thelDH status of the glioma cells.

Another interesting biological sample is the cerebrospinal fluid, wherein a sufficient amount of tumoral DNA is available for determining the IDH status of the glioma.

Furthermore, when the process is implemented on the basis of blood or cerebrospinal fluid of a patient with glioma, the parameter "telomere length" cannot be used: indeed, these samples contain different types of cells, and the measure of the telomere length status concern all cells of the sample. Therefore the telomere length status of the glioma only cannot be determined. It is to be noted, however, that this measure of telomere length status can be performed anyway on the sample, even if results will not be used in the classification process of the invention.

In this embodiment, the *in vitro* process for classifying a glioma comprises the following steps:
a. Measuring at least, from said blood sample or cerebrospinal fluid sample from a glioma patient, the Alternative Lengthening of Telomeres (ALT) status of said glioma;
b. Optionally, determining the isocitrate dehydrogenase genes mutation status (IDH status) of said glioma;
c. Based on the data obtained in steps (a) and optionally (b), classifying said glioma either in two (a) or four (steps a and b) groups as detailed below.

If the ALT status and IDH status are known (a and b) the four following classes are "available": oligodendroglioma -like, glioblastoma IDHwt -like, Astrocytoma_IDHmt (grade II to IV), and other gliomas.

If only step (a) is performed, glioma are classified either:
- in the C-circle positive group (encompassing Astrocytoma grade II - III, GBM_IDHmt and high grade ALT+ tumors) or
- in the C-circle negative/Other group (encompassing the other gliomas and part of ALT+ glioma without circulating tumoral cells)

Figure 12B illustrates these results, where the glioma are classified in two groups CC POS and Other.

In this embodiment of the invention, usually no solid glioma sample is available. Therefore, the histological grading of the tumor is not available. The process is therefore limited to the classification in two classes, without any further sub-classification of the gliomas according to their histological grade II or III.

In a specific embodiment of the invention, the process of the invention comprises both steps (a) and (c).

In a specific embodiment of the invention, the process of the invention comprises the three steps (a), (b) and (c).

In a specific embodiment of the invention, the process of the invention comprises a further step of determination of the TERT status (mutated or wild-type) of the glioma.

In a specific embodiment of the invention, the process of the invention consists in both steps (a) and (c).

In a specific embodiment of the invention, the process of the invention consists in three steps (a), (b) and (c).

In a specific embodiment of the invention, at least two glioma patient biological samples obtained at different time points are submitted to said process of classification of the glioma, for a follow-up of the patient over time. Indeed, the implementation of the process on a blood sample is particularly advantageous for the following up over time of a patient having a glioma, in particular for detecting the evolution or recurrence after treatment of said glioma.

### Telomere maintenance mechanism (TMM)

In vertebrates, chromosome extremities, designed as telomeres, consist of:
- a repeated motif of sequence 5'-(TTAGGG)n-3', that is 10-15 kilobases long in human at birth, and a 3' G-rich single-stranded tail of 150 to 300 bases;
- six associated proteins which comprise the sheltering complex that promotes telomere protection, by ensuring stability and assisting specialized replication machinery for accurate extension of chromosome ends and recruitment of telomerase. The six proteins comprise three DNA-binding poteins (TRF1, TRF2, POT1) interconnected by three additional proteins (TIN2, ACD, RAP1).

Telomeres play vital roles in eukaryotic cells, in particular by limiting DNA replication and therefore avoiding unlimited cell proliferation, via their gradual shortening at each replicative cycle.

Telomerase is a ribonucleic reverse transcriptase enzyme, able to add the telomere repeat sequence to the 3' end of telomeres. It consists of a catalytic subunit called TERT (for Telomerase Reverse Transcriptase), an essential RNA component TERC that functions as the RNA template for the addition of the telomeric repeats, and a series of auxiliary components.

In most tumour cells, the TMM is linked to the reactivation of telomerase. Nevertheless, about 10% of tumour cells acquire immortality through the telomerase-independent alternative lengthening of telomeres (ALT) mechanism.

Glioma can therefore be classified as "ALT-dependent tumors", when the telomerase-independent mechanism is in place, and "ALT-independent tumors" when a telomerase-dependant mechanism is activated in the cell.

Within the ALT-dependent group, different subgroup can be identified: ALT intermediate, ALT+ and ALT ++.

The ALT-independent tumors define the ALT- subgroup, wherein no C circle is detected.

Any suitable assay for determining the ALT status of cells can be used for the implementation of the process.

Henson *et al.* (2009) have disclosed an assay based on partially single-stranded telomeric (CCCTAA)n DNA circles that allows a specific measurement of the ALT status. This assay is known as "the C-circle assay" or "CC assay".

Briefly, this C-circle assay can be summarized as follow:
Telomeric extrachromosomal DNA (ECT) is particularly enriched in ALT-dependant cells versus telomerase-positive or mortal cells. Among different species of ECT, C-circles is a specific and sensitive marker of the ALT process. A C-circle is composed of a circular DNA of C₃TA₂ sequence, partially double stranded with a short T₂AG₃ sequence. The technique of Rolling Circle Amplification (RCA) has been used to detect telomeric circles in ALT+ cells. The "CC-assay" involves the use of ϕ29, a highly processive DNA polymerase that is auto-primed by the partial G-strand (TTAGGG)n.

The amplified telomeric DNA is then quantified together with the genomic telomeric DNA after and before incubation with ϕ29. A ratio between these two quantities of DNA is calculated, and compared to the same ratio obtained in telomerase positive cells and in ALT-dependant cells. (Henson et al., 2017).

The amplified telomeric DNA can be quantified by all techniques well known by the man skilled in the art, such as hybridization and flow cytometry methods.

In a preferred embodiment of the invention, the quantification of the amplified telomeric DNA circles is performed as described in (Gil & Coetzer, 2004) with a real-time polymerase chain reaction (PCR) - based method, and particularly with a telomere-specific PCR.

A "C-circle" value, expressed in arbitrary units, equal to the following ratio:
Quantity of total telomeric DNA (including RCA-amplifiedC-circles) / Quantity of total telomeric DNA (without amplification of C-circle content) is measured by the means described above for each glioma sample.

This value is then compared to at least three thresholds values, previously defined according to the general knowledge of the man skilled in the art.

In particular, the inventors have defined four thresholds as follow:
- A threshold said "high";
- A threshold said "intermediate"
- A threshold said "positive"
- A threshold said "negative".

In a specific embodiment of the invention, in the *in vitro* process, the ALT status in (a) is determined by performing a C-circle assay coupled to a telomere-specific PCR, thereby obtaining a C-circle value and selecting the ALT status of the glioma from:
- ALT++, when the C-circle value is greater than the threshold value "high";
- ALT+, when the C-circle value is greater than the threshold value "positive";
- ALT intermediate, when the C-circle value is less than the threshold value "intermediate", and
- ALT-, when the C-circle value is less than the threshold value "negative".

In a specific embodiment of the invention, the *in vitro* process for classifying a glioma comprises the following steps:
a. Measuring at least, from a glioma patient biological sample, the Alternative Lengthening of Telomeres (ALT) status of said glioma;
b. Optionally, determining the isocitrate dehydrogenase genes mutation status (IDH status) of said glioma;
c. Based on the data obtained in steps (a) and optionally (b) and, if available, the histological grade of said glioma, classifying said glioma in one of the five following classes: oligodendroglioma -like, glioblastoma IDHwt -like, glioblastoma IDHmt -like, low-grade astrocytoma -like, and other gliomas,
wherein the ALT status is measured by performing a C-circle assay coupled to a telomere-specific PCR, thereby obtaining a C-circle value and selecting the ALT status of the glioma.

### Telomere length status

Measurement of telomere length is another important parameter of the process, measured in step (a) in certain implementation of the process of the invention.

The original method for determining telomere length employed Southern hybridization to determine the mean terminal restriction fragment length. However this technique was labor-intensive, time-consuming and necessitates high amount of DNA (over 1µg), not available in standard clinical use. Various alternative techniques have since been proposed, including slot blots, next generation sequencing, hybridization protection assays and flow cytometry.

Although any technique for determining the telomere length status might be employed in the implementation of the process of the invention, in a preferred embodiment, telomere length status in step (a) is determined by quantifying the telomeric DNA with a telomere-specific PCR.

In particular, the real-time quantitative PCR named TeloPCR described in (Gil & Coetzer, 2004) can be used for this quantification.

This TeloPCR has been adapted into a duplex TeloPCR, both amplifying the housekeeping gene (using a fluorescent hydrolysis probe) and the telomeric sequences (SybrGreen).

According to this embodiment, the quantification technique comprises the following steps:
- Quantitative PCR reactions are initiated with addition of primers specifics for telomeres (TeloPCR) and for the used housekeeping gene(s) such as 36B4 (36B4 PCR) to samples of total DNA;
- The efficiency of TeloPCR (E_{Telo}) and 36B4 (E_{36B4}) PCR is determined on the basis of standard curves generated from two reference DNA samples, one ALT+ and one ALT-; for example, a DNA from previously characterized ALT+ tumors and ALT- tumors, either extracted from paraffin block or frozen sample;
- Products of the qPCR are quantified as well-known by the man skilled in the art, following the E_{Telo}^{- *CT(telo)*} / E_{36B4}^{- *CT(36B4)*} method, where the threshold cycle (CT) is the cycle at which the fluorescence level reaches a certain amount (the threshold), determined by the second derivative method, for each qPCR (CT(Telo) and CT36B4), and E stands for PCR efficiency and is determined for each gene according to the standard curves.

The telomere content value representative of the mean quantity of telomeric sequences in cells for each sample is obtained. This value is next normalized by internal controls obtained from DNA extracted from ALT+ and ALT- cell lines (such as U2OS and HeLa, respectively), as it is well known by the man skilled in the art. The resulting value is designated as "T-length" value.

Four threshold have been defined as a function of increasing TL : short, middle-long, long and very long. With these thresholds, the telomere length status of the glioma can be determined as follow:
- Long telomere, when the T-length value is greater than the threshold value "very long";
- Intermediate telomere, when the T-length value is comprised between the threshold values "middle-long" and "long"; and
- Short telomere, when the T-length value is less than the threshold value "short

In a specific embodiment of the invention, the *in vitro* process for classifying a glioma comprises the following steps:
a. Measuring at least, from a glioma patient biological sample, the Alternative Lengthening of Telomeres (ALT) status of said glioma;
b. Optionally, determining the isocitrate dehydrogenase genes mutation status (IDH status) of said glioma;
c. Based on the data obtained in steps (a) and optionally (b) and, if available, the histological grade of said glioma, classifying said glioma in one of the five following classes: oligodendroglioma -like, glioblastoma IDHwt -like, glioblastoma IDHmt -like, low-grade astrocytoma -like, and other gliomas,
wherein the telomere length status in step (a) is measured by quantifying the telomeric DNA with a telomere-specific PCR, thereby obtaining a T-length value and selecting the telomere length status of the glioma.

In a specific embodiment of the invention, the ALT status and the T-length status are measured concomitantly with the same experimentation, comprising:
- the C-circle assay, and
- a telomere specific quantitative PCR.

Specifically, both substeps of step (a) are concomitantly performed by one duplex PCR as presented above.

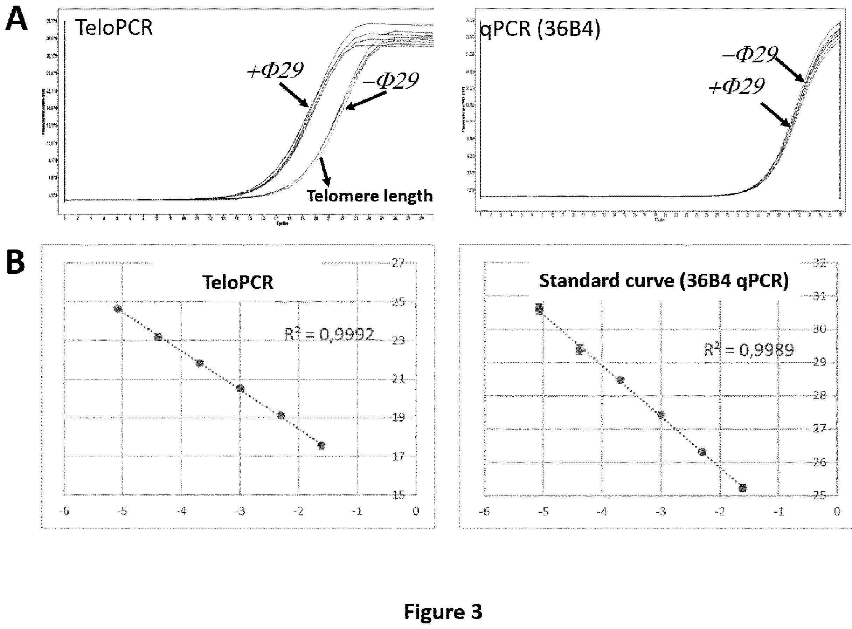

Figure 3 presents an example of standard curves that can be used for the calculation of the qPCR efficiency, and that are useful for determining both the C-circle value and the telomere length.

The present invention also relates to a process for determining the Telomere Maintenance Mechanisms status (TMM status) of any tumor, comprising the measuring, from a patient biological sample, of both the Alternative Lengthening of Telomeres (ALT) status and the telomere length status of said tumor, wherein the ALT status is measured by performing a C-circle assay coupled to a telomere-specific PCR, thereby obtaining a C-circle value and selecting the ALT status of the tumor from:
- ALT++, when the C-circle value is greater than the threshold value "high";
- ALT+, when the C-circle value is greater than the threshold "positive";
- ALT-, when the C-circle value is less than the threshold value "positive";
wherein the telomere length status is measured by quantifying the telomeric DNA with a telomere-specific PCR, thereby obtaining a T-length value and selecting the telomere length status of the tumor from:
- Long telomere, when the T-length value is greater than the threshold value "very long";
- Intermediate telomere, when the T-length value is comprised between the threshold values "very long" and "middle-long"; and
- Short telomere, when the T-length value is less than the threshold value "short",
and wherein both measures are concomitantly performed by one duplex PCR.

### Threshold values

In a specific embodiment of the invention, the values of the parameters "ALT status" and, where appropriate, the telomere length status, are measured and then compared each one to at least one threshold value.

For the purposes of carrying out the above process, the term "threshold value" is intended to mean a value determined with a group of glioma samples that are well characterized, and whose ALT status and telomere length status have been previously determined.

Based on the results obtained for these groups of specific gliomas, inventors have defined threshold values, also designed as cut-off values, for ALT status and telomere length that are defined below.

Other threshold values can be easily determined by those skilled in the art by means of their general knowledge.

Therefore, the value obtained for the ALT status is compared to at least one ALT threshold value, in order to determine the ALT status of the glioma; and the value obtained for the TL status is compared to at least one T-length threshold value, in order to determine the telomere length (TL) status of the glioma.

Thresholds and classification may evolve during the implementation of the process, and in particular may be function of the nature of the tested biological sample.

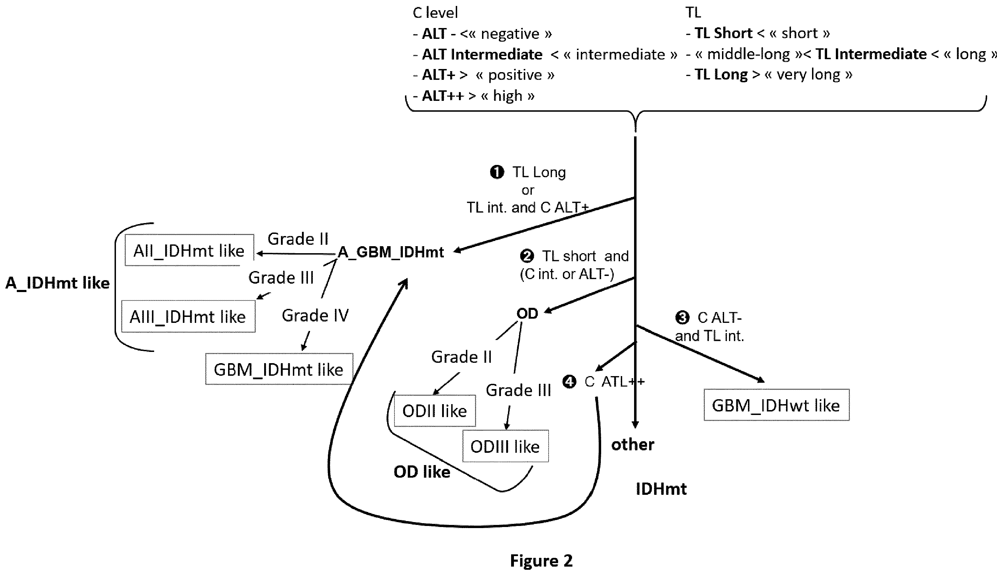

Figures 1 and 2 illustrate a first algorithm according to the invention, based on four different levels of C circles. Results of this implementation of the classification process are presented in figures 4 and 5.

Recently, a novel classification, more precise, has been defined with the implementation of the process according to the invention on a higher number of biological samples.

The second algorithm designated as "Telo-DIAG" is illustrated in figure 9, and results are presented in figures 11, 12 and 13.

This specific implementation of the process "Telo-DIAG" is described in details below :
The thresholds for the C-circle (CC) level are the following for FFPE samples;
   - ALT++/ high threshold :CC over 2
   - ALT intermediate : CC over 1.24 (between 1.24 and 2)
   - ALT negative : CC below 1.24
The thresholds for the C-circle (CC) level are the following for frozen samples;
   - ALT++/ high threshold : CC over 1.3
   - ALT negative : CC below 1.3
The thresholds for the telomere length (TL) status are the following for FFPE samples: - TL Long: TL > 4
   - TL short: TL < 2.6
   - TL intermediate : TL over 1.1 and below 4 (1.1< TL< 4)

The thresholds for the telomere length (TL) status are the following for frozen samples :
- TL Long: TL > 1.8
- TL short: TL < 1.2

The TMM categories are defined as follow for FFPE samples:
- ALT+ for tumors either ALT++ or TL long, or concomitantly ALT intermediate and TL intermediate
- ALT- for tumors concomitantly ALT negative and TL short (most of OD show even shorter telomeres and are enriched for values of TL below 1.5)

The TMM categories are defined as follow for frozen samples:
- ALT+ for tumors either ALT++ or TL long,
- ALT- for tumors concomitantly TL short and ALT negative.

On the basis of the TMM status, one can define the TeloDIAG as follow :
- tLGA : ALT+ with IDH mutation and grade II to III
- tGBM_IDHmt : ALT+ with IDH mutation and grade IV
- t_A-IV : ALT+ without IDH mutation (assimilated to grade IV tumors)
- tOD : ALT- with IDH mutation
- tGBM : ALT- without IDH mutation

This specific classification process takes into account high grade glioma without mutation in IDH such as pontine gliomas (with or without histone mutation) and includes the classification of IDHwt astrocytoma that are now considered as grade IV in respect with the Impact-now update 3.

### IDH status

The parameter of IDH status has previously been described for the classification of gliomas.

In a specific embodiment of the invention, the IDH status in optional step (b) is determined by sequencing both genes encoding proteins IDH1 and IDH2.

Sequences of human isocitrate dehydrogenase proteins 1 and 2 are well known of the man skilled in the art, and can be found in proteins database such as GeneBank and OMIM.

Wild-type sequence of human IDH1 is represented in SEQ ID NO:1, and wild type sequence of human IDH2 is represented in SEQ ID NO: 2, as presented in table 1 below:

| | | |
|---|---|---|
| SEQ ID NO: 1 | | UniProtKB - O75874, OMIM 147700 |
| SEQ ID NO: 2 | | UniProtKB - P48735, OMIM 147650 |

The IDH status of the glioma cells is determined as follows:
- The glioma is said of "IDH mutated (IDHmt) status" in case of the identification of a point mutation of residue R132 in IDH1 protein, and/or of a point mutation of residue R172 in IDH2 protein,
   and
- The glioma is said of "IDH wild-type (IDHwt) status" in case of each of both proteins IDH1 and IDH2 present a wild-type sequence at the respective residues R132 and R172.

Step (b) of the process according to the invention is optional. Indeed, and as illustrated in figure 1, determination of the IDH status is particularly relevant in the following case: when the intermediate classification (after step (a)) is "GbmOD" i.e. an oligodendroglioma or a glioblastoma; indeed, a glioma presenting the following features:
∘ ALT status = ALT-; and
∘ T-length = intermediate ;
could be an oligodendroglioma or a glioblastoma.

In this case, a step (b) of determination of the IDH status is necessary for the final classification, where:
o If IDH status = IDHwt, then the glioma is a primary glioblastoma (GBM_IDHwt or GBMI);
o If IDH status = IDHmt, the glioma is an oligodendroglioma (OD), with a much better prognostic.

### Histological grading of the glioma

Advantageously, the tumor grading has been previously determined on the basis of immuno-histochemical analyses of pictures of said tumor, thereby selecting the tumor grade of the glioma from stage II, III or IV. The histological grade of the glioma is said to be available.

These grades are defined in the actual WHO classification of gliomas, and are based on the presence of the following criteria of the tumor cells: mitosis, vascular proliferation and necrosis.

The grade of the glioma will advantageously be determined by a man skilled in the art such as a neuro-specialized physician.

When no solid glioma sample is available, for example in the case of the implementation of the process on glioma DNA extracted from blood or cerebrospinal fluid of the patient, the histological grading of the tumor is not available and therefore not known, since it has not been determined.

### Reclassification of a glioma

As previously presented, the subclass of "astrocytoma IDHwt" as determined by WHO's classification is particularly heterogeneous and difficult to classify.

Often, these gliomas are not actually conventional "astrocytomas" as defined by the WHO classification.

Therefore, the median overall survival is highly variable with a median of 2.5 years, which is much more severe than for classical astrocytoma. Moreover, therapeutic options are not defined by guidelines and might be wrongly chosen.

Therefore, there is a need for reclassifying these specific gliomas, as well as unclassified gliomas called "others" or "NEC", with a novel combination of parameters representative of the nature of said glioma.

In a particular aspect, the invention concerns the present *in vitro* process of classification, wherein a glioma of the class "astrocytoma" with a IDH status "IDHwt", or a glioma classified "other" or "NEC", is re-classified in one of the following classes: oligodendroglioma-like, glioblastoma IDHwt-like, glioblastoma IDHmt-like, t-low grade Astrocytoma (tLGA) and t-Astrocytoma grade IV (tA-IV), and other gliomas.

In this process, the histological grade of the "astrocytoma IDHwt" is usually known before the implementation of the process, and therefore is available. However, optionally, the histological grade of the "astrocytoma IDHwt" might be determined during the process.

Although the IDH status of this kind of glioma is IDHwt, the glioma might be re-classified in a class such as "glioblastoma_IDHmt-like" or "low-grade astrocytoma -like", although tumors in this class present an IDHmt status. Nevertheless, it is understood that in this case, the glioma previously classified as "IDHwt astrocytoma" keeps its status of IDHwt, but is categorized in this astrocytoma class because the patient affected with said glioma would present the same median overall survival than those affected with such astrocytoma.

In this implementation of the process, further steps could be included such as determination of the status of TERT (mutated or wild-type) (Vinagre et al., 2013) and determination of the status of ATRX (present or absent) (Nandakumar et al., 2017).

### Uses of the process of the invention in therapy

The present invention also relates to a process for choosing a therapeutic strategy for treating a glioma, comprising the steps of:
a) Implementing the *in vitro* process for classifying said glioma as presented above, and
b) Based on said classification, choosing the more adapted therapeutic strategy for the patient affected by said glioma.

These therapies can be the actual "gold standard" therapies: radiotherapy with a concomitant or adjuvant chemotherapy, with chemotherapeutic agents such as Temozolomide or PCV, a combination treatment for brain tumors including procarbazine, lomustine, and vincristine.

The present invention also relates to a process for adapting a therapeutic strategy for treating a glioma, comprising the steps of:
a) Implementing the *in vitro* process for classifying said glioma as presented above, and
b) Based on said classification, adapting the therapeutic strategy for the patient affected by said glioma.

A promising therapeutic approach for the treatment of gliomas is the administration of inhibitors of any telomere maintenance mechanism. In particular, it has been shown that telomerase inhibitors increase the response to radiotherapy in a murine orthotopic model of human gliobastoma (Ferrandon *et al.,* 2015).

Thus, the present invention relates to an inhibitor of at least one telomere maintenance mechanism (TMM) for its use in the treatment of a glioma, wherein said glioma has been previously classified according to the process of the invention.

Advantageously, the determination of the ALT status of the glioma allows the practician to choose between TMM inhibitors specific of the ALT mechanism, or specific of the telomerase reactivation mechanism.

### Implementation of the process with a computer

In a specific implementation of these processes, data obtained in steps (a) (b) and (c) are recorded on a computer device, into a software program that is configured to memorize said data and to execute steps to classify the glioma in function of said parameters data.

Accordingly, the present invention also concerns a computer program product comprising code instructions for implementing a process as described above, for classifying a glioma.

### Kit for the implementation of the processes

The present invention also concerns a kit for the implementation of the processes as described, comprising:
- Reagents suitable for performing a C-circle assay;
- Reagents suitable for performing a duplex Telomere-specific PCR; and
- Adequate internal controls comprising genomic DNA from ALT+ cells and from ALT- cells,
wherein both substeps of measure of C-circle and telomere length are concomitantly performed by one duplex PCR.

In particular, internal controls are chosen among cell lines expressing the telomerase (ALT- cells), such as breast cancer-derived cells (HeLa), and among cell lines using the process of "Alternative lengthening of telomere" (ALT+ cells), such as osteosarcoma cell lines.

Other useful internal controls can be also present in said kit for the implementation of the processes of the invention.

### EXAMPLES

Although the present invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

### Example 1. C-circle reaction

Rolling circle amplification of C-circle is performed as described in (Henson *et al.,* 2009) and (Henson *et al.,* 2014). Briefly, 3.2µl of total genomic DNA (5ng/µL) were incubated for 18h at 30°C with 3,75 units of φi29 DNA polymerase (New England Biolabs) (0.375µL of 10U/µL), in 0.2µg/µL of BSA, 0.1% Tween, 4µM DTT (Dithiothreitol), 1mM dNTP, 1µL of 10X NEB buffer. Enzyme is heat-inactivated at 65°C for 20 min. The same reaction is performed without the enzyme φ29 (φ-).

For each experiment, two internal controls are added, namely TA and ALT. TA and ALT correspond to total genomic DNA extracted from HeLa (ALT-) and U2OS (ALT+) cell lines respectively.

The 10µL of φ- and φ+ reactions are then diluted by adding 30µL of water (molecular biology grade), 5µL are used to performed each qPCR reaction.

### Example 2. qPCR experiment

### • TeloPCR

Oligonucleotides for the qPCR reaction have been previously described in (Gil *et al.,* 2004) and (Lau *et al.,* 2013) and are listed below

The sequence for oligonucleotides used in the qPCR are presented in table 2:

| | | |
|---|---|---|
| SEQ ID NO: 3 | 5'-CGGTTTGTTTGGGTTTGGGTTTGGGTTTGGGTTTGGGTT-3' | forward Tel1a |
| SEQ ID NO: 4 | 5'-GGCTTGCCTTACCCTTACCCTTACCCTTACCCTTACCCT-3' | reverse primer tel2B |
| SEQ ID NO: 5 | 5'-CAGCAAGTGGGAAGGTGTAATCC-3' | RPL0/36B 4 forward primer |
| SEQ ID NO: 6 | 5'-CCATTCTATCATCAACGGGTACAA-3' | RPL0/36B 4 reverse primer |

Telo-PCR and qPCR against 36B4 are run in duplicate for each condition φ- and φ+, on a 480 Light Cycler Thermocycler (Roche, Houwald, Luxembourg), in 1X final LightCycler^{®} DNA Master SYBR Green I (10µL), 200nM final of TeloPCR-specific primers or 300 nMm final of 36B4-specific primers. Details of thermocycling conditions are detailed below

For each qPCR, the exact conditions are summarized in tables 3 and 4 below:

**Table 3. TeloPCR**

| Analysis Mode | Cycles | Segment | Target Température | Hold time | Acquisition Mode |
|---|---|---|---|---|---|
| Pre Incubation | | | | | |
| None | 1 | | 95°C | 10 min | none |

| Amplification | | | | | |
|---|---|---|---|---|---|
| Quantification | 32 | Denaturation | 95°C | 5s | none |
| | | Annealing | 56 °C Rate = 4°C/s | 10 s | none |
| | | Extension | 72°C | 60 s | single |

| Melting Curve | | | | | |
|---|---|---|---|---|---|
| Melting Curves | 1 | Denaturation | 95 °C | 0 s | none |
| | | Annealing | 65 °C | 15s | none |
| | | Melting | 95 °C Ramp Rate = 0,1 °C/s | 0 s | continuous |

| Cooling | | | | | |
|---|---|---|---|---|---|
| none | 1 | | 40 °C | 30 s | none |

**Table 4. PCR 36B4**

| Analysis Mode | Cycles | Segment | Target Température | Hold time | Acquisition Mode |
|---|---|---|---|---|---|
| Pre Incubation | | | | | |
| None | 1 | | 95°C | 10 min | none |

| Amplification | | | | | |
|---|---|---|---|---|---|
| Quantification | 36 | Denaturation | 95°C | 5s | none |
| | | Annealing | 58 °C | 10s | none |
| | | Extension | 72°C | 40 s | single |

| Melting Curve | | | | | |
|---|---|---|---|---|---|
| Melting Curves | 1 | Denaturation | 95 °C | 0 s | none |
| | | Annealing | 65 °C | 15s | none |
| | | Melting | 95 °C Ramp Rate = 0,1 °C/s | 0 s | continuous |

| Cooling | | | | | |
|---|---|---|---|---|---|
| none | 1 | | 40 °C | 30 s | none |

### • dTeloPCR

Oligonucleotides for the amplification of telomeric sequences have been previously described in (Gil *et al.,* 2004) and (Lau *et al.,* 2013). The oligonucleotides and the probe targeting RPLP0 have been customed-designed and are described below.

The sequences of oligonucleotides used in the qPCR are presented in table 5:

| | | |
|---|---|---|
| SEQ ID NO: 3 | 5'-CGGTTTGTTTGGGTTTGGGTTTGGGTTTGGGTTTGGGTT-3' | forward Tel1a |
| SEQ ID NO: 4 | 5'-GGCTTGCCTTACCCTTACCCTTACCCTTACCCTTACCCT-3' | reverse primer tel2B |
| SEQ ID NO: 7 | 5'- AGC AAG TGG GAA GGT GTA ATC-3' | RPL0/36B 4 forward primer |
| SEQ ID NO: 8 | 5'-CCA TTC TAT CAT CAA CGG GTA CA-3' | RPL0/36B 4 reverse primer |
| SEQ ID NO: 9 | 5'-5Cy/CAC AGA CAA/ TAO/GGC CAG GAC TCG TT/31AbRQSp- 3' 5Cy and 31AbRQSp are fluorescent compounds, TAO is an internal quencher | RPLP0/36 B4 probes |

Duplex Telo-PCR is run in duplicate for each condition φ- and φ+, on a 480 Light Cycler Thermocycler (Roche, Houwald, Luxembourg), in 1X final LightCycler^{®} DNA Master SYBR Green I (10µL), 0.4µM final of TeloPCR-specific primers or 1µM final of 36B4-specific primers and 0.22µM of 36B4 probes. Details of thermocycling conditions are detailed in table 6 below.

**Table 6. Conditions for duplex TeloPCR**

| Analysis Mode | Cycles | Segment | Target Température | Hold time | Acquisition Mode |
|---|---|---|---|---|---|
| Pre Incubation | | | | | |
| None | 1 | | 95°C | 10 min | none |

| Amplification | | | | | |
|---|---|---|---|---|---|
| Quantification | 32 | Denaturation | 95°C | 5s | none |
| | | Annealing | 56 °C Rate = 4°C/s | 10 s | none |
| | | Extension | 72°C | 60 s | single |

| Melting Curve | | | | | |
|---|---|---|---|---|---|
| Melting Curves | 1 | Denaturation | 95 °C | 0 s | none |
| | | Annealing | 65 °C | 15s | none |
| | | Melting | 95 °C Ramp Rate = 0,1 °C/s | 0 s | continuous |

| Cooling | | | | | |
|---|---|---|---|---|---|
| none | 1 | | 40 °C | 30 s | none |

### Example 3. Data analysis, normalization and classification

The fluorescence in logarithmic scale is analyzed as a function of PCR cycle, the threshold is determined by the second derivative method (all experiments). Intersection between the threshold of amplification curve gives the CT for each reaction. The fluorescence chanel corresponding to the TeloPCR is the following : SYBR Green (465-510). For the dTeloPCR, two channels are analyzed : SYBR Green (465-510) for the telomeric sequence and CY5 (618-660) for RPLP0/36B4.

Efficiency of TeloPCR (Eₜₑₗₒ) and 36B4 (E_{36B4}) PCR are respectively 1.70111 and 1.9672. For each reaction, the following value is calculated : Eₜₑₗₒ^{-CT}/ E_{36B4}^{-CT} , and annotated as φ+ and φ- as a function of the initial circle reaction.
φ- correspond to the telomere length (T-Length)
Cᵣ correspond to the Circle score and is calculated as follow : φ+ / φ-

The difference of T-Length between the two internal controls (U2OS and HeLa) Δ is calculated and correspond to x units (determined for each batch of controls) with Δ= φ-HeLa - φ-_{U2OS}
TLₙₒᵣₘ is then calculated for each sample as follow : φ-/(Δ/x)

A standard curve for calculation of qPCR efficiency is presented in figure 3.

As shown in figure 1, the first step (a) of the algorithm allows the classification of the tumors into 4 main categories:
- OD (oligodendroglioma),
- GbmOD (glioblastoma (GBM_IDHwt in figure 1) or OD),
- A_GBM_IDHmt (AD (All_IDHmt in figure 1), AA (Alll_IDHmt in figure 1), or GBMII (GBM_IDHmt in figure 1), and
- others.

GbmOD category is then subclassified in step (b) according to the IDH status:
- If the tumor is IDHwt : GBM_IDHwt or
- If the tumor is IDHmt: OD.

All glioma are then classified according to their grade in step (c). In particular, tumors of the categorie "A_GBM_IDHmt" will be subclassified, according to their grade, into All-IDHmt like (AD), Alll_IDHmt like (AA) or GBM_IDHmt like (GBMII).

An example of equations is given below; however, the indicated numbers have been defined for this experiment only, and are not limitating the scope of the claimed invention.

An example of equations is given below; however, the indicated numbers have been defined for this experiment only, and are not limitating the scope of the claimed invention.

### 1^{st} step (a)

A=(SI(TLₙₒᵣₘ>4;"A_GBMII";SI(ET(Cᵣ>1,5;TLₙₒᵣₘ>0,8);"A_GBMII";SI(ET(TLₙₒᵣₘ<0,66; Cᵣ<1,9);"OD";SI(ET(Cᵣ <1,28;ET(TLₙₒᵣₘ>0,66; TLₙₒᵣₘ<3));"GbmOD":SI(C, >2;"A_GBMII";"other"))))))
2^{nd} step (b) with IDH status (MT or WT)
B=SI(ET(A="GbmOD";IDH="MT");"OD";(SI(ET(A="GbmOD";IDH="WT");"GBM";A)))
3^{rd} step with grading G (II, III, IV)
C=SI(ET(B="A_GBMII";G="III");"AA";SI(ET(B="A_GBMII";G="II");"AD";SI(ET(B="A_GBM II";G="IV");"GBMII";B)

Alternatively, if using the dTeloPCR method, as shown in figure 10, the first step (TMM) of the algorithm allows the classification of the tumors into 3 main categories:
- ALT+ : AA_GBMII
- ALT- : GBM_OD or GBM
- Others (not interpretable)

TMM Categories are then subclassified in the second step (Telostep1) according to the IDH status:
- If the tumor is GBM or GBM_OD and IDHwt : tGBM or
- If the tumor is GBM or GBM_OD and IDHmt: tOD.
- If the tumor is AA_GBMII and IDHmt : IDHmt_AA_GBMII
- If the tumor is AA_GBMII and IDHwt : AA_GBMII

All glioma are then classified according to their grade in the third step. In particular, tumors of the categorie "IDHmt_AA_GBMII" will be subclassified, according to their grade, into tLGA for grade II and III, or into tGBM_IDHmt for grade IV.

An example of equations is given below; the indicated thresholds are function of the nature of the sample, and have been defined for this experiment only.
- **dTeloPCR associated algorithm**
   **∘ For FFPE sample :**

### TMM class

A=(SI(OU(TLₙₒᵣₘ >4; Cᵣ >2);"AA_GBMII";SI(ET(Cᵣ >=1,24; TLₙₒᵣₘ >1,1);"AA_GBMII";SI(ET(TLₙₒᵣₘ <1,5; Cᵣ <1,24);"GBM_OD";SI(ET(Cᵣ <1,24;ET(TLₙₒᵣₘ >=1,5; TLₙₒᵣₘ <2,6));"GBM";"other")))))

### Telostep1 (use of the factor IDH1/2 mutated = MT or wild type =WT)

B=SI(ET(OU(A="GBM_OD";A="GBM");|IDH="MT");"tOD";SI(ET(OU(A="GBM_OD";A="G BM");IDH="WT");"tGBM";SI(ET(A="AA_GBMII";IDH="MT");"IDHmt_AA_GBMII";A)))

### TeloDIAG (use of the histological grading II, III or IV)

C=SI(B="IDHmt_AA_GBMII";SI(OU(grading="II";grading="III");"tLGA";"tGBM_IDHmt");SI (B="AA_GBMII";"tAIV":B))
**o For frozen sample :**

### TMM class

A=(SI(ET(TLₙₒᵣₘ <1,2; Cᵣ<=1,3); "GBM_OD"; SI(Cᵣ>1,3;"AA_GBMII"; SI(TLₙₒᵣₘ>1,8;"AA_GBMII";"autre"))))
The following steps : TElostep1 and TeloDIAG are the same as for FFPE samples
   **o For Circulating DNA**
A=SI(Cᵣ >1,25;"AA_GBMII";"other")

### Example 4. Obtained results with the TeloPCR method

First, only 180 patients with molecular biomarkers in agreement with the immuno-histochemical classification (i.e. with the exception of anaplastic astrocytoma IDHwt) were analyzed.

These gliomas have been classified according to the standard algorithm according to the WHO 2016 classification (upper line), or with the algorithm of the invention (bottom line) and are presented in figure 4A.

The algorithm of the invention allows a separation into five subclasses: "oligodendroglioma-like", "glioblastoma IDHwt-like", "glioblastoma IDHmt-like", "low-grade astrocytoma -like" and "other" (the code is depicted in the legend).

Both processes of classification are concordant for 93.5% of gliomas. However for 3.8% of gliomas, the classfications are different (noted misclassified). With the algorithm of the invention, 2.7% of gliomas are classified as "others".

In conclusion, with the classification process according to the invention:
∘ For 94% of the gliomas, the classification was the same with the standard algorithm than with the process of the invention;
∘ 3.8% of the gliomas were differently classified (noted "misclassified") - their re-classification was relevant with the OS and DFS curves presented in figure 5;
∘ 2.7% of the gliomas are classified in the category "others".

Figure 4B shows the obtained results when considering only the 29 gliomas with discordant molecular biomarkers and immuno-histochemical classification.

In this case, the classification obtained with the algorithm of the invention is in agreement with the standard process of classification for 72% of gliomas.

The figure 5 presents a comparison of the curves of median overall survival (OS) and median disease-free survival (DFS) obtained after standard classification and classification according to the process of the invention

The overall survival (OS, upper curves) and the disease free survival (DFS, bottom curves) have been followed for each patient.

210 patients were analyzed, classified as follow according to the standard classification: 74 low-grade astrocytoma, 24 glioblastoma IDHwt, 57 glioblastoma IDHmt, and 55 oligodendroglioma.

Among these patients, 29 have discordant molecular parameters as regard with the immuno-histological classification.

The algorithm of the invention allows a separation into five subclasses: oligodendroglioma-like, glioblastoma IDHwt-like, glioblastoma IDHmt-like, low-grade astrocytoma -like (A_IDHmt like), and other gliomas.

Note that the curves are highly similar regarding the overall survival, and that the algorithm is even more discriminant that the standard classification considering the low grade astrocytoma and the oligodendroglioma.

### Example 5. Comparison of duplex Telo-PCR versus radio-activity detection of C-circle.

The C-circle assay combine a first step of rolling circle amplification of circular DNA (partially double stranded), and a second step wherein the telomeric DNA is quantified. The technic of reference proceeds through an hybridization of amplified telomeric DNA by radioactive probe. The quantification of the radio-active signal is proportional to the CC content in a linear manner.

To validate the duplex TeloPCR (dTeloPCR) as a detection mode, independent RCA assays have been run, either coupled with dTelo-PCR or with radio-activity detection on 120 DNA extracted from frozen tumors (Fig. 6A) and on 37 DNA extracted from total blood samples (Fig.6B). Cutoffs of 7 AU (arbitraty Unit) and 1.25 for dTelo-PCR were used as positive threshold. 97% and 87% of concordant results were observed, respectively for blood and frozen tumors. Regarding tumors, the radio-active method is concordant with the ATRX and TERT status (N=98 samples) for 90% of the tumors (8 false negative and 2 false positives results). The concordance reaches 96% for the dTelo-PCR (3 false negative and 1 false positive).

Considering the blood sample (Fig.6B), C-circle were detected, probably due to circulating tumoral cells (CTC). The two technics are concordant in 92% of cases (N=37), with results in agreement with the ATRX and TERT status (N=34) in 85% for radioactive detection (5 false negative) and 79% (7 false negative) for the dTeloPCR.

False negative/positives results are probably in relation with samples of poor quality and/or deleterious storage conditions.

These results show that the dTeloPCR detection method is concordant with the radio-active detection method.

### Example 6. Identification of the telomeric parameters in frozen and FFPE (Formalin-Fixed Paraffin-Embedded) samples, by using the dTeloPCR method

152 DNA from frozen gliomas were analyzed (Fig.7B) and 161 from FFPE tumors (Fig.7A) to determine:
(i) if C-circle can be detected in DNA extracted from FFPE sample, and
(ii) if the telomeric parameters determined by dTeloPCR can be combined to improve the detection of ALT+ sample in both types of samples.

Control DNA from a TERT positive, ALT- cell line HeLa or ALT positive cell line U2OS were used as internal controls in each experiment (N=40 independent determination), results are shown in Fig. 7C.

The C-circle (CC) rate is effectively positive (over 0 in logarithmic values, Fig.7) in astrocytoma and GBM-IDHmt in both types of samples (frozen and FFPE). A higher telomeric sequence content (TL) is observed in these tumors, as compared with oligodendroglioma and GBM.

By comparing frozen and FFPE samples, a higher TL but a lower CC level were noticed in FFPE sample in ALT+ samples.

To confirm this observation, DNA from paired frozen and FFPE samples was extracted from 16 different tumors; a higher rate of CC and a lower TL in frozen sample was confirmed (Fig. 8A).

Formaldehyde fixative is known to induce DNA single and double strand breaks due to oxidative lesions. Break in C-circle impedes the RCA reaction, which would explain a lower CC rate. Moreover, fragmentation of long double-stranded-telomeric sequences (over 5kb) would favor TeloPCR reaction by increasing the content of DNA matrix and by producing shorter but more numerous amplicons, thereby biasing the TeloPCR reaction toward an increase in the TL.

A long conservation of FFPE specimen before extraction of DNA is expected to decrease the CC level. All DNA samples analyzed herein have been extracted during the months following the inclusion in FFPE.

To test this hypothesis, additional DNA extractions from 21 FFPE samples from ALT+tumors, 3 to 8 years after inclusion, have been realized. Results are shown in Fig. 8B. Indeed, a huge decrease into the CC amount is observed for the major part of tumors. Among the 21 samples, two (from 4 and 3 years) turned to be below the C-circle threshold of 1.23 in the newly made extraction and would have been false negative.

Using the results of Fig.7, combinations of thresholds of CC and TL have been determined to assign a "TMM status": either ALT+ or ALT-.

Figure 9 presents schematically the algorithm of the invention.

The concordance of this TMM classification was validated in regard to the presence of TERT mutation for ALT- subgroup, and in regard to the loss of ATRX for the ALT+ subgroup. This is in particular shown in Fig. 10A and 10C.

When taking into account the 321 gliomas, even if molecular markers are missing, all of the A and GBM-IDHmt tumors were detected as ALT+, and all of the OD and GBM (classically TERT positive) were classified as ALT- (Fig.10A). Only five samples were classified as "other", in link with discordant CC and TL parameters, probably due to a low quality/quantity of DNA.

The TMM status of 96 tumors with discordant molecular and immuno-histochemical parameters was determined and are shown on Fig. 10B and 10D, such as ATRX deleted GBM, 1p19q not deleted OD, IDHwt astrocytomas or tumors with incoherent telomeric parameters (TERTmt and ATRXloss or TERTwt and ATRXwt).

Regarding the 60 tumors with no telomeric markers (TERTwt and ATRXwt), 19 ALT+ tumors (9 GBM, 8 GBM_IDHmt and 2 OD) were identified. Half of the double positive (TERTmt and ATRXloss) tumors were classified as ALT+ (6 Astrocytoma, 1 GBM and 1 GBM-IDHmt).

It is thus possible to assign a "TMM status" even in discordant tumors, and this is of great help for the classification and therefore for diagnosis.

### Example 7. Diagnosis value of the TeloDiag, algorithm of the invention (dTEloPCR method)

The C-circle assay results (CC and TL) have been combined with the IDH1/2 status and the histological grading, to create a simple algorithm "TELODiag" to assign 5 glioma subtypes: this algorithm is schematically represented on Fig.9.

The five subtypes of glioma are the following:
- tOD (ALT-, IDHmt), grade II or III
- tLGA (ALT +, IDHmt), grade II or III
- tGBM-IDHmt (ALT+, IDHmt), grade IV
- tA_IV (ALT+, IDHwt), any grades
- tGBM (ALT-, IDHwt), grade IV

The classification of glioma obtained on the basis of the algorithm TeloDiag was compared to the WHO 2016 classification (taking into account the impactnow updates), first in concordant tumors (Fig.11 A, B) and next with discordant tumor (Fig.11 C, D).

311 tumors over the 321 tested were classified as expected with the WHO classification, five are attributed to a different groups (5 were unclassified, "other").

Regarding the discordant tumors, 51 were classified on the same corresponding WHO section, the other 45 were classified in a different subgroup.

### Example 8. Detection of CC in blood sample by the dTeloPCR method

Total blood cells from 208 patients were collected by centrifugation and subjected to CC assay.

As expected, the TL reflecting the length of telomere in the blood nucleated cells were unchanged among the different glioma subgroups (Fig.12A). However, CC were detected in some of the blood samples from patients with ALT+ tumors (Astrocytoma and GBM-IDHmt) (Fig.12A). This result is in agreement with the presence of a few circulating tumoral cells (CTC). If setting 1.25 as a cutoff of CC to determine the ALT status, we retrieved C-circle in 57% of the blood samples from patient with an ALT+ tumors (Fig.12. B,C). However 6% (N=5) of blood sample corresponding to OD are considered as false positive values. These results are under investigation, to determine the error risk.

Modalities of blood collection, CTC preparation and conservation are under investigation to maximize the detection of C-circle.

We have analyzed103 DNA extracted from buffy coat and obtained only 12% of positive result regarding patients with ALT+ tumors. Thus a total blood centrifugation of 1000g 10min should be operate to collect CTC. The delay between surgery and blood sampling has to be the lowest as possible, however we detected CC in blood drawed months after surgery (maybe due to relapse).

### CONCLUSION

We have established a new easy, fast and low-cost assay, based on qPCR detection, able to determine the TMM on frozen and FFPE glioma samples. We established a new diagnosis tool, the "TeloDiag", an algorithm combining the TMM, the IDH mutation and the histological grading.

This classification gives concordant results with the reference WHO 2016 classification with a refined number of prognosis groups, and could be helpful in tumors with discordant molecular and histological feature, as is presented in figure 13.

We also detected ALT+ circulating cells in blood from 57% of patients with an ALT+ tumors. This is of importance : (i) to secure the initial diagnosis in some critical situations (radio-necrosis, differential diagnosis...) and to avoid deleterious biopsy, (ii) to follow the treatment response of ALT+ tumors, without invasive analysis.

### REFERENCES

WO 2017/127803
1. cIMPACT-NOW update 4: diffuse gliomas characterized by MYB, MYBL1, or FGFR1 alterations or BRAFV600E mutation. - PubMed - NCBI. https://www.ncbi.nlm.nih.gov/pubmed/?term=PMID%3A+30848347.
2. cIMPACT-NOW update 3: recommended diagnostic criteria for "Diffuse astrocytic glioma, IDH-wildtype, with molecular features of glioblastoma, WHO gr... - PubMed - NCBI. https://www.ncbi.nlm.nih.gov/pubmed/?term=PMID%3A+30259105.
3. cIMPACT-NOW update 2: diagnostic clarifications for diffuse midline glioma, H3 K27M-mutant and diffuse astrocytoma/anaplastic astrocytoma, IDH-mutant. - PubMed - NCBI. https://www.ncbi.nlm.nih.gov/pubmed/?term=PMID%3A+29497819.
4. cIMPACT-NOW update 1: Not Otherwise Specified (NOS) and Not Elsewhere Classified (NEC). - PubMed - NCBI. https://www.ncbi.nlm.nih.gov/pubmed/?term=PMID%3A+29372318.
5. McDonald KL, McDonnell J, Muntoni A, Henson JD, Hegi ME, von Deimling A, Wheeler HR, Cook RJ, Biggs MT, Little NS, Robinson BG, Reddel RR, Royds JA. Presence of alternative lengthening of telomeres mechanism in patients with glioblastoma identifies a less aggressive tumor type with longer survival. J Neuropathol Exp Neurol. 2010 Jul;69(7):729-36
6. Nguyen DN, Heaphy CM, de Wilde RF, Orr BA, Odia Y, Eberhart CG, Meeker AK, Rodriguez FJ. Molecular and morphologic correlates of the alternative lengthening of telomeres phenotype in high-grade astrocytomas. Brain Pathol. 2013 May;23(3):237-43.
7. Mangerel J, Price A, Castelo-Branco P, Brzezinski J, Buczkowicz P, Rakopoulos P, Merino D, Baskin B, Wasserman J, Mistry M, Barszczyk M, Picard D, Mack S, Remke M, Starkman H, Elizabeth C, Zhang C, Alon N, Lees J, Andrulis IL, Wunder JS, Jabado N, Johnston DL, Rutka JT, Dirks PB, Bouffet E, Taylor MD, Huang A, Malkin D, Hawkins C, Tabori U. Alternative lengthening of telomeres is enriched in, and impacts survival of TP53 mutant pediatric malignant brain tumors. Acta Neuropathol. 2014 Dec;128(6):853-62.
8. (Fogli A, Demattei MV, Corset L, Vaurs-Barrière C, Chautard E, Biau J, Kémény JL, Godfraind C, Pereira B, Khalil T, Grandin N, Arnaud P, Charbonneau M, Verrelle P. Detection of the alternative lengthening of telomeres pathway in malignant gliomas for improved molecular diagnosis. J Neurooncol. 2017 Nov;135(2):381-390.
9. Hakin-Smith V, Jellinek DA, Levy D, Carroll T, Teo M, Timperley WR, McKay MJ, Reddel RR, Royds JA. Alternative lengthening of telomeres and survival in patients with glioblastoma multiforme. Lancet. 2003 Mar 8;361(9360):836-8.
10. Louis DN, Perry A, Reifenberger G, von Deimling A, Figarella-Branger D, Cavenee WK, Ohgaki H, Wiestler OD, Kleihues P, Ellison DW. The 2016 World Health Organization Classification of Tumors of the Central Nervous System: a summary. Acta Neuropathol. 2016 Jun;131(6):803-20. doi: 10.1007/s00401-016-1545-1. Epub 2016 May 9. Review.
11. Henson JD, Cao Y, Huschtscha LI, et al. DNA C-circles are specific and quantifiable markers of alternative-lengthening-of-telomeres activity. Nat. Biotechnol. 2009;27(12):1181-1185.
12. Henson JD, Lau LM, Koch S, et al. The C-Circle Assay for alternative-lengthening-of-telomeres activity. Methods San Diego Calif. 2017;114:74-84.
13. Gil ME, Coetzer TL. Real-time quantitative PCR of telomere length. Mol. Biotechnol. 2004;27(2):169-172.
14. Inda MM, Fan X, Muñoz J, Perot C, Fauvet D, Danglot G, Palacio A, Madero P,Zazpe I, Portillo E, Tuñón T, Martínez-Peñuela JM, Alfaro J, Eiras J, Bernheim A,Castresana JS. Chromosomal abnormalities in human glioblastomas: gain in chromosome 7p correlating with loss in chromosome 10q. Mol Carcinog. 2003 Jan;36(1):6-14. PubMed PMID: 12503074.
15. Vinagre J, Almeida A, Pópulo H, Batista R, Lyra J, Pinto V, Coelho R, Celestino R, Prazeres H, Lima L, Melo M, da Rocha AG, Preto A, Castro P, Castro L, Pardal F, Lopes JM, Santos LL, Reis RM, Cameselle-Teijeiro J, Sobrinho-Simões M, Lima J, Máximo V, Soares P. Frequency of TERT promoter mutations in human cancers. Nat Commun. 2013;4:2185.
16. Nandakumar, P., Mansouri, A., & Das, S. The Role of ATRX in Glioma Biology. Frontiers in oncology, 7, 236 (2017).
17. Ferrandon S, Malleval C, El Hamdani B, et al. Telomerase inhibition improves tumor response to radiotherapy in a murine orthotopic model of human glioblastoma. Mol Cancer. 2015;
18. Lau LM, Dagg RA, Henson JD, Au AY, Royds JA, Reddel RR. Detection of alternative lengthening of telomeres by telomere quantitative PCR. Nucleic Acids Res. 2013 Jan;41(2):e34.

## Claims

1. An *in vitro* process for classifying a glioma, comprising the following steps:
a. Measuring at least, from a glioma patient biological sample, the Alternative Lengthening of Telomeres (ALT) status of said glioma ;
b. determining the isocitrate dehydrogenase genes mutation status (IDH status) of said glioma;
c. Based on the data obtained in steps (a) and (b) and, on the histological grade of said glioma, classifying said glioma in one of the five following classes: oligodendroglioma-like, glioblastoma IDHwt-like, glioblastoma IDHmt-like, low-grade astrocytoma-like, and other gliomas,
wherein the class "low-grade astrocytoma-like" comprises two subclasses designated as t-low grade Astrocytoma (tLGA) and t-Astrocytoma grade IV (tA-IV),
wherein the ALT status in (a) is measured by performing a C-circle assay coupled to a telomere-specific PCR, both substeps being concomitantly performed by one duplex PCR, thereby obtaining a C-circle value expressed in arbitrary units, equal to the following ratio: Quantity of total telomeric DNA including RCA-amplified C-circles / Quantity of total telomeric DNA without amplification of C-circle content,
and selecting the ALT status of the glioma from:
- ALT++, when the C-circle value is greater than the threshold value "high";
- ALT+, when the C-circle value is greater than the threshold "positive";
- ALT-, when the C-circle value is less than the threshold value "positive",
Wherein the threshold value is intended to mean a value determined with a group of glioma samples that are well characterized and whose ALT status and telomere length status have been previously determined and wherein furthermore:
• a glioma of the class "oligodendroglioma-like" (tOD) is **characterized by** the following data: (a) ALT-, (b) IDH mutated, and (c) histological grade II or III;
• a glioma of the class "glioblastoma IDHwt-like" (tGBM) is **characterized by** the following data: (a) ALT-, (b) IDH wild-type, and histological grade IV,
• a glioma of the class "glioblastoma IDHmt-like" (tGBM-IDHm) is **characterized by** the following data: (a) ALT+, (b) IDH mutated, and (c) histological grade IV,
• a glioma of the subclass "t-low-grade astrocytoma" (tLGA) is **characterized by** the following data: (a) ALT+, (b) IDH mutated, and (c) histological grade II or III,
• a glioma of the subclass "t-astrocytoma grade IV" (tA IV) is **characterized by** the following data: (a) ALT+, (b) IDH wild-type, and (c) any histological grade.

2. The *in vitro* process according to claim 1, wherein each class of glioma is defined by a median time of overall survival of the patients affected by said glioma.

3. The *in vitro* process according to anyone of claims 1 to 2, wherein the steps (a) and (b) are realized on the basis of DNA extracted from a glioma sample that has been conserved in paraffin or frozen.

4. The *in vitro* process according to claim 3, wherein the step (a) further comprises the measure of the telomere length status of said glioma.

5. The *in vitro* process according to claim 4, wherein the telomere length status in (a) is measured by quantifying the telomeric DNA with a telomere-specific PCR, thereby obtaining a T-length value and selecting the telomere length status of the glioma from:
- Long telomere, when the T-length value is greater than the threshold value "very long";
- Intermediate telomere, when the T-length value is comprised between the threshold values "very long" and "middle-long"; and
- Short telomere, when the T-length value is less than the threshold value "short".

6. The *in vitro* process according to anyone of claims 1 to 5, wherein the IDH status in step (b) is determined by sequencing both genes encoding proteins IDH1, whose wild-type sequence is represented in SEQ ID NO.1, and IDH2, whose wild-type sequence is represented in SEQ ID NO. 2, thereby selecting the IDH status of the glioma from:
- IDH mutated (IDHmt) status in case of a mutation of residue R132 in IDH1 protein, and/or a mutation of residue R172 in IDH2 protein, or
- IDH wild-type (IDHwt) status in case of both proteins IDH1 and IDH2 present a wild-type sequence at the respective residues R132 and R172.

7. The *in vitro* process according to anyone of claims 4 to 6, wherein:
• A glioma of the class "oligodendroglioma-like" (tOD) is **characterized by** the following data: (a) ALT- and short telomere, (b) IDH mutated, and (c) histological grade II or III;
• a glioma of the class "glioblastoma IDHwt-like" (tGBM) is **characterized by** the following data: (a) ALT- and short telomere, (b) IDH wild-type, and histological grade IV,
• a glioma of the class "glioblastoma IDHmt-like" (tGBM-IDHm) is **characterized by** the following data: (a) (ALT++ or long telomere) or (ALT+ and intermediate telomere), (b) IDH mutated, and (c) histological grade IV,
• a glioma of the subclass "t-low-grade astrocytoma" (tLGA) is **characterized by** the following data: (a) (ALT++ or long telomere) or (ALT+ and intermediate telomere), (b) IDH mutated, and (c) histological grade II or III,
• a glioma of the subclass "t-astrocytoma grade IV" (tA IV) is **characterized by** the following data: (a) (ALT++ or long telomere) or (ALT+ and intermediate telomere), (b) IDH wild-type, and (c) histological grade IV.

8. The *in vitro* process according to anyone of claims 1 to 7, wherein a glioma of the class "astrocytoma" with a IDH status "IDHwt", or a glioma classified "other" is re-classified in one of the following classes: oligodendroglioma-like, glioblastoma IDHwt-like, glioblastoma IDHmt-like, t-low grade Astrocytoma (tLGA) and t-Astrocytoma grade IV (tA-IV), and other gliomas.

9. The *in vitro* process according to anyone of claims 1 to 2 or 6 to 8, wherein the patient biological sample is a blood sample or a cerebrospinal fluid sample.

10. The *in vitro* process according to claim 9, wherein the TERT status of the glioma is further determined.

11. The *in vitro* process according to claim 9 or 10, wherein at least two patient biological samples obtained at different time points are submitted to said process of classification of the glioma, for a follow-up of the patient over time.

12. Use of a kit for the implementation of the process according to claim 1, said kit comprising:
- Reagents suitable for performing a C-circle assay;
- Reagents suitable for performing a Telomere-specific PCR; and
- Adequate internal controls comprising genomic DNA from ALT+ cells and from ALT- cells,
wherein both substeps of measure of C-circle and telomere length are concomitantly performed by one duplex PCR.

## Patentansprüche

1. In-vitro-Verfahren zur Klassifizierung eines Glioms, das die folgenden Schritte umfasst:
a. Messen, von einer biologischen Probe eines Gliom-Patienten, mindestens des Status der Alternativen Verlängerung von Telomeren (ALT) des Glioms;
b. Bestimmen des Status der Mutation *der* Isocitrat-Dehydrogenase-Gene (IDH-Status) des Glioms;
c. auf der Grundlage der in den Schritten (a) und (b) erhaltenen Daten und des histologischen Grads des Glioms, Klassifizieren des Glioms in eine der fünf folgenden Klassen: Oligodendrogliom-ähnlich, Glioblastom-IDHwt-ähnlich, Glioblastom-IDHmt-ähnlich, Low-Grade-Astrocytom-ähnlich oder andere Gliome,
wobei die Klasse "Low-Grade-Astrocytom-ähnlich" zwei Unterklassen umfasst, die als t-Low-Grade-Astrocytom (tLGA) und t-Astrocytom Grad IV (tA-IV) bezeichnet werden,
wobei der ALT-Status in (a) durch Durchführen eines C-Circle-Assays, gekoppelt an eine Telomer-spezifische PCR gemessen wird, wobei beide Teilschritte gleichzeitig durch eine Duplex-PCR durchgeführt werden, wodurch ein C-Circle-Wert, der in beliebigen Einheiten ausgedrückt ist, gleich dem folgenden Verhältnis erhalten wird: Menge der gesamten telomerischen DNA einschließlich RCAamplifizierter C-Circle / Menge der gesamten telomerischen DNA ohne Amplifizierung des C-Circle-Gehalts;
und Auswählen des ALT-Status des Glioms von:
- ALT++, wenn der C-Circle-Wert größer als der Schwellenwert "hoch" ist;
- ALT+, wenn der C-Circle-Wert größer als der Schwellenwert "positiv" ist;
- ALT-, wenn der C-Circle-Wert kleiner als der Schwellenwert "positiv" ist,
wobei der Schwellenwert dazu bestimmt ist, einen Wert zu bedeuten, der mit einer Gruppe von Gliom-Proben bestimmt wird, die gut charakterisiert sind und deren ALT-Status und Telomer-Längenstatus vorhergehend bestimmt wurden und wobei ferner:
• ein Gliom der Klasse "Oligodendrogliom-ähnlich" (tOD) durch die folgenden Daten gekennzeichnet ist: (a) ALT-, (b) IDH-mutiert und (c) histologischer Grad II oder III;
• ein Gliom der Klasse "Glioblastom-IDHwt-ähnlich" (tGBM) durch die folgenden Daten gekennzeichnet ist: (a) ALT-, (b) IDH-Wildtyp und histologischer Grad IV,
• ein Gliom der Klasse "Glioblastom-IDHmt-ähnlich" (tGBM-IDHm) durch die folgenden Daten gekennzeichnet ist: (a) ALT+, (b) IDH-mutiert, und (c) histologischer Grad IV,
• ein Gliom der Unterklasse "t-Low-Grade-Astrocytom" (tLGA) durch die folgenden Daten gekennzeichnet ist: (a) ALT+, (b) IDH-mutiert und (c) histologischer Grad II oder III,
• ein Gliom der Unterklasse "t-Astrocytom Grad IV" (tA IV) durch die folgenden Daten gekennzeichnet ist: (a) ALT+, (b) IDH-Wildtyp und (c) ein beliebiger histologischer Grad.

2. In-vitro-Verfahren nach Anspruch 1, wobei jede Gliom-Klasse durch eine mediane Gesamtüberlebenszeit der von dem Gliom betroffenen Patienten definiert ist.

3. In-vitro-Verfahren nach einem der Ansprüche 1 bis 2, wobei die Schritte (a) und (b) auf der Grundlage von DNA ausgeführt werden, die von einer Gliom-Probe extrahiert wurde, die in Paraffin konserviert oder eingefroren war.

4. In-vitro-Verfahren nach Anspruch 3, wobei der Schritt (a) ferner das Messen des Telomer-Längenstatus des Glioms umfasst.

5. In-vitro-Verfahren nach Anspruch 4, wobei der Telomer-Längenstatus in (a) durch Quantifizieren der telomerischen DNA mit einer Telomer-spezifischen PCR gemessen wird, wodurch ein T-Längenwert erhalten wird und der Telomer-Längenstaus des Glioms ausgewählt wird von:
- langes Telomer, wenn der T-Längenwert größer als der Schwellenwert "sehr lang" ist;
- intermediäres Telomer, wenn der T-Längenwert zwischen den Schwellenwerten "sehr lang" und "mittellang" enthalten ist; und
- kurzes Telomer, wenn der T-Längenwert kleiner als der Schwellenwert "kurz" ist.

6. In-vitro-Verfahren nach einem der Ansprüche 1 bis 5, wobei der IDH-Status in Schritt (b) bestimmt wird durch Sequenzieren beider Gene, die für die Proteine IDH1, deren Wildtyp-Sequenz in der SEQ ID NR. 1 dargestellt ist, und IDH2 kodieren, deren Wildtyp-Sequenz in der SEQ ID NR. 2 dargestellt ist, und dadurch Auswählen des IDH-Status des Glioms von:
- IDH-mutierter (IDHmt) Status im Fall einer Mutation des Rests R132 im IDH1-Protein und/oder Mutation des Rests R172 im IDH2-Protein, oder
- IDH-Wildtyp (IDHwt) Status im Fall, in dem beide Proteine IDH1 und IDH2 eine Wildtyp-Sequenz an den jeweiligen Resten R132 und R172 aufweisen.

7. In-vitro-Verfahren nach einem der Ansprüche 4 bis 6, wobei:
• ein Gliom der Klasse "Oligodendrogliom-ähnlich" (tOD) durch die folgenden Daten gekennzeichnet ist: (a) ALT- und kurzes Telomer, (b) IDH-mutiert und (c) histologischer Grad II oder III;
• ein Gliom der Klasse "Glioblastom-IDHwt-ähnlich" (tGBM) durch die folgenden Daten gekennzeichnet ist: (a) ALT- und kurzes Telomer, (b) IDH-Wildtyp und histologischer Grad IV,
• ein Gliom der Klasse "Glioblastom-IDHmt-ähnlich" (tGBM-IDHm) durch die folgenden Daten gekennzeichnet ist: (a) (ALT++ oder langes Telomer) oder (ALT+ und intermediäres Telomer), (b) IDH-mutiert, und (c) histologischer Grad IV,
• ein Gliom der Unterklasse "t-Low-Grade-Astrocytom" (tLGA) durch die folgenden Daten gekennzeichnet ist: (a) (ALT++ oder langes Telomer) oder (ALT+ und intermediäres Telomer), (b) IDH-mutiert und (c) histologischer Grad II oder III,
• ein Gliom der Unterklasse "t-Astrocytom Grad IV" (tA IV) durch die folgenden Daten gekennzeichnet ist: (a) (ALT++ oder langes Telomer) oder (ALT+ und intermediäres Telomer), (b) IDH-Wildtyp und (c) histologischer Grad IV.

8. In-vitro-Verfahren nach einem der Ansprüche 1 bis 7, wobei ein Gliom der Klasse "Astrocytom" mit einem IDH-Status "IDHwt" oder ein Gliom, das als "andere" klassifiziert ist, in eine der folgenden Klassen neu klassifiziert wird: Oligodendrogliom-ähnlich, Glioblastom-IDHwt-ähnlich, Glioblastom-IDHmt-ähnlich, t-Low-Grade-Astrocytom (tLGA) und t-Astrocytm Grad IV (tA-IV) und andere Gliome.

9. In-vitro-Verfahren nach einem der Ansprüche 1 bis 2 oder 6 bis 8, wobei die biologische Patientenprobe eine Blutprobe oder eine Cerebrospinalflüssigkeitsprobe ist.

10. In-vitro-Verfahren nach Anspruch 9, wobei ferner der TERT-Status des Glioms bestimmt wird.

11. In-vitro-Verfahren nach Anspruch 9 oder 10, wobei mindestens zwei biologische Patientenproben, die an unterschiedlichen Zeitpunkten erhalten werden, dem Verfahren zur Klassifizierung des Glioms für eine Nachsorge des Patienten über die Zeit unterzogen werden.

12. Verwendung eines Kits zur Implementierung des Verfahrens nach Anspruch 1, wobei der Kit umfasst:
- Reagenzien, die sich zum Durchführen eines C-Circle-Assays eignen;
- Reagenzien, die sich zum Durchführen einer Telomer-spezifischen PCR eignen; und
- geeignete interne Kontrollen, die genomische DNA von ALT+-Zellen und von ALT--Zellen umfassen,
wobei beide Teilschritte des Messens von C-Circle und Telomerlänge gleichzeitig durch eine Duplex-PCR durchgeführt werden.

## Revendications

1. Procédé *in vitro* de classification d'un gliome, comprenant les étapes suivantes :
a. mesurer, à partir d'un échantillon biologique provenant d'un patient atteint d'un gliome, le statut ALT (Alternative Lengthening of Telomeres) dudit gliome ;
b. déterminer le statut de mutation des gènes de l'isocitrate déshydrogénase (statut IDH) dudit gliome ;
c. Sur la base des données obtenues aux étapes (a) et (b) et du grade histologique dudit gliome, classer ledit gliome dans l'une des cinq classes suivantes : type oligodendrogliome, type glioblastome IDHwt, type glioblastome IDHmt, type astrocytome de bas grade et autres gliomes,
dans lequel la classe « de type astrocytome de bas grade » comprend deux sous-classes désignées comme t-astrocytome de bas grade (tLGA) et t-astrocytome de grade IV (tA-IV),
dans lequel le statut ALT est mesuré en (a) en effectuant un test C-circle couplé à une PCR spécifique des télomères, les deux sous-étapes étant réalisées simultanément par une PCR duplex, ce qui permet d'obtenir une valeur C-circle exprimée en unités arbitraires, égale au rapport suivant : quantité totale d'ADN télomérique comprenant les C-circles amplifiés par RCA / quantité totale d'ADN télomérique sans amplification du contenu en C-circles,
et sélectionner le statut ALT du gliome parmi :
- ALT++, lorsque la valeur du C-circle est supérieure à la valeur seuil « élevée » ;
- ALT+, lorsque la valeur du C-circle est supérieure au seuil « positif » ;
- ALT-, lorsque la valeur du C-circle est inférieure à la valeur seuil « positive »,
la valeur seuil désignant une valeur déterminée à partir d'un groupe d'échantillons de gliomes bien caractérisés dont le statut ALT et la longueur des télomères ont été préalablement déterminés, et dans lequel, en outre :
• un gliome de la classe « de type oligodendrogliome » (tOD) est **caractérisé par** les données suivantes : (a) ALT-, (b) IDH muté, et (c) grade histologique II ou **III** ;
• un gliome de la classe « de type glioblastome IDHwt » (tGBM) est **caractérisé par** les données suivantes : (a) ALT-, (b) IDH de type sauvage, et grade histologique IV,
• un gliome de la classe « glioblastome de type IDHmt » (tGBM-IDHm) est **caractérisé par** les données suivantes : (a) ALT+, (b) IDH muté, et (c) grade histologique IV,
• un gliome de la sous-classe « t-astrocytome de bas grade » (tLGA) est **caractérisé par** les données suivantes : (a) ALT+, (b) IDH muté, et (c) grade histologique II ou III,
• un gliome de la sous-classe « t-astrocytome de grade IV » (tA IV) est **caractérisé par** les données suivantes : (a) ALT+, (b) IDH de type sauvage, et (c) tout grade histologique.

2. Procédé *in vitro* selon la revendication 1, dans lequel chaque classe de gliome est définie par une durée médiane de survie globale des patients atteints dudit gliome.

3. Procédé *in vitro* selon l'une quelconque des revendications 1 à 2, dans lequel les étapes (a) et (b) sont réalisées sur la base d'ADN extrait d'un échantillon de gliome qui a été conservé dans de la paraffine ou congelé.

4. Procédé *in vitro* selon la revendication 3, dans lequel l'étape (a) comprend en outre la mesure de l'état de la longueur des télomères dudit gliome.

5. Procédé *in vitro* selon la revendication 4, dans lequel l'état de la longueur des télomères en (a) est mesuré en quantifiant l'ADN télomérique à l'aide d'une PCR spécifique des télomères, ce qui permet d'obtenir une valeur de longueur T et de sélectionner l'état de la longueur des télomères du gliome parmi :
- Télomère long, lorsque la valeur de longueur T est supérieure à la valeur seuil « très long » ;
- Télomère intermédiaire, lorsque la valeur de longueur T se situe entre les valeurs seuils « très long » et « moyen-long » ; et
- Télomères courts, lorsque la valeur de longueur T est inférieure à la valeur seuil « court ».

6. Procédé *in vitro* selon l'une quelconque des revendications 1 à 5, dans lequel le statut IDH à l'étape (b) est déterminé par séquençage des deux gènes codant pour les protéines IDH1, dont la séquence de type sauvage est représentée par SEQ ID NO. 1, et IDH2, dont la séquence de type sauvage est représentée par SEQ ID NO. 2, sélectionnant ainsi le statut IDH du gliome parmi :
- un statut IDH muté (IDHmt) en cas de mutation du résidu R132 dans la protéine IDH1 et/ou d'une mutation du résidu R172 dans la protéine IDH2, ou
- un statut IDH de type sauvage (IDHwt) dans le cas où les deux protéines IDH1 et IDH2 présentent une séquence de type sauvage au niveau des résidus respectifs R132 et R172.

7. Procédé *in vitro* selon l'une quelconque des revendications 4 à 6, dans lequel :
• Un gliome de la classe « de type oligodendrogliome » (tOD) est **caractérisé par** les données suivantes : (a) ALT- et télomères courts, (b) IDH mutée, et (c) grade histologique II ou III ;
• un gliome de la classe « de type glioblastome IDHwt » (tGBM) est **caractérisé par** les données suivantes : (a) ALT- et télomères courts, (b) IDH de type sauvage, et grade histologique IV,
• un gliome de la classe « glioblastome de type IDHmt » (tGBM-IDHm) est **caractérisé par** les données suivantes : (a) (ALT++ ou télomères longs) ou (ALT+ et télomères intermédiaires), (b) mutation de l'IDH, et (c) grade histologique IV,
• un gliome de la sous-classe « t-astrocytome de bas grade » (tLGA) est **caractérisé par** les données suivantes : (a) (ALT++ ou télomères longs) ou (ALT+ et télomères intermédiaires), (b) IDH muté, et (c) grade histologique II ou **III,**
• un gliome de la sous-classe « t-astrocytome de grade IV » (tA IV) est **caractérisé par** les données suivantes : (a) (ALT++ ou télomère long) ou (ALT+ et télomère intermédiaire), (b) IDH de type sauvage, et (c) grade histologique IV.

8. Procédé *in vitro* selon l'une quelconque des revendications 1 à 7, dans lequel un gliome de la classe « astrocytome » présentant un statut IDH « IDHwt », ou un gliome classé « autre », est reclassé dans l'une des classes suivantes : de type oligodendrogliome, de type glioblastome IDHwt, de type glioblastome IDHmt, t-astrocytome de bas grade (tLGA) et t-astrocytome de grade IV (tA-IV), et autres gliomes.

9. Procédé *in vitro* selon l'une quelconque des revendications 1 à 2 ou 6 à 8, dans lequel l'échantillon biologique du patient est un échantillon de sang ou un échantillon de liquide céphalo-rachidien.

10. Procédé *in vitro* selon la revendication 9, dans lequel le statut TERT du gliome est en outre déterminé.

11. Procédé *in vitro* selon la revendication 9 ou 10, dans lequel au moins deux échantillons biologiques du patient obtenus à des moments différents sont soumis audit procédé de classification du gliome, pour un suivi du patient au cours du temps.

12. Utilisation d'un kit pour la mise en œuvre du procédé selon la revendication 1, ledit kit comprenant :
- des réactifs adaptés à la réalisation d'un test C-circle ;
- des réactifs adaptés à la réalisation d'une PCR spécifique des télomères ; et
- des contrôles internes adéquats comprenant de l'ADN génomique provenant de cellules ALT+ et de cellules ALT-,
dans lequel les deux sous-étapes de mesure du C-circle et de la longueur des télomères sont réalisées simultanément par une PCR duplex.
